# EUROPEAN PATENT SPECIFICATION

(11) **EP 1 959 762 B1**
(45) Date of publication and mention of the grant of the patent: **08.10.2014**
(21) Application number: 06837991.6
(22) Date of filing: 17.11.2006
(51) Int. Cl.: A23L 1/236, A23L 1/30, A23L 1/303, A23L 1/305, A23L 2/38, A23L 2/52, A23L 2/60

(54) **HIGH-POTENCY SWEETENER COMPOSITION FOR TREATMENT AND/OR PREVENTION OF AUTOIMMUNE DISORDERS, COMPOSITIONS AND BEVERAGES SWEETENED THEREWITH**
SÜSSMITTELZUSAMMENSETZUNG MIT HOHER SÜSSKRAFT FÜR DIE BEHANDLUNG UND/ODER VORBEUGUNG VON AUTOIMMUNERKRANKUNGEN, UND HIERMIT GESÜSSTE ZUSAMMENSETZUNGEN UND GETRÄNKE
COMPOSITION D'EDULCORANT TRES PUISSANT POUR LE TRAITEMENT ET/OU LA PREVENTION DE TROUBLES AUTO-IMMUNS ET COMPOSITIONS SUCREES AVEC CELLE-CI

(30) Priority: 23.11.2005 US 739302 P; 23.11.2005 US 739124 P; 19.06.2006 US 805209 P; 19.06.2006 US 805216 P; 02.11.2006 US 556118
(43) Date of publication of application: 27.08.2008
(73) Proprietor: THE COCA-COLA COMPANY, Atlanta GA 30313 (US)
(72) Inventor: PRAKASH, Indra, Alpharetta, Georgia 30022 (US); DUBOIS, Grant, E., Roswell, Georgia 30076 (US)
(74) Representative: Bittner, Thomas L.
(86) International application number: PCT/US2006/044797
(87) International publication number: WO 2007/061907

(56) References cited:
- EP-A- 0 862 864
- EP-A- 1 210 880
- EP-A1- 0 657 169
- WO-A-01/28357
- AU-A- 5 654 000
- CN-A- 1 084 703
- CN-A- 1 440 691
- CN-A- 1 644 109
- JP-A- 4 135 460
- JP-A- 8 256 725
- JP-A- 55 088 675
- JP-A- 56 055 174
- JP-A- 60 075 252
- JP-A- 60 098 968
- JP-A- 60 188 035
- JP-A- 63 304 964
- JP-A- 2000 236 842
- JP-A- 2003 246 729
- US-A- 5 962 678
- US-A1- 2002 132 037
- SCHIFFMAN, S. ET AL.: "Investigation of synergism in binary mixtures of sweeteners" BRAIN RESEARCH BULLETIN, vol. 38, no. 2, 1995, pages 105-120, XP002428872
- DATABASE WPI Week 197747 Derwent Publications Ltd., London, GB; AN 1977-83952Y XP002457253 -& JP 52 122676 A (MORITA KAGAKU KOGYO KK) 15 October 1977 (1977-10-15)
- DATABASE WPI Week 197906 Derwent Publications Ltd., London, GB; AN 1979-10896B XP002457254 -& JP 53 148575 A (SAN-EI CHEM IND LTD) 25 December 1978 (1978-12-25)
- TADHANI, M. AND SUBHASH, R.: "Preliminary studies on Stevia rebaudiana leaves: proximal composition, mineral analysis and phytochemical screening" J. MED. SCI., vol. 6, no. 3, 2006, pages 321-326, XP002458119

## Description

### FIELD OF THE INVENTION

The present invention relates generally to a functional sweetener, a functional sweetened composition, a method for imparting a more sugar-like temporal profile, more sugar-like flavor profile or both to a functional sweetener composition or functional sweetened composition, and a functional beverage comprising the functional sweetened composition.

### BACKGROUND OF THE INVENTION

Nutrition usually focuses on the relationship between food and human health from the perspective of ensuring all essential nutrients are adequately supplied and utilized to optimize health and well being. As diseases typically related to nutritional deficiency were managed, there has been a recognition that many nutrients have health benefits beyond basic nutrition. Accordingly, functional ingredients have been identified as playing a key role in an individual's overall health.

"Functional ingredients" offer potential health benefits beyond basic nutrition when incorporated into foods, beverages, and other orally ingested products. Such ingredients have been shown to help reduce the risk of or manage a number of health concerns, including cancer, heart and cardiovascular disease, gastrointestinal health, menopausal symptoms, osteoporosis, and vision. Since 1993, the United States Food and Drug Administration (FDA) has approved numerous health claims for the labeling of food products with information related to the health benefits of functional food (U.S. Food and Drug Administration, A Food Labeling Guide (2000)).

| **Functional Food** | | **Health Benefit** | |
|---|---|---|---|
| ▪ | Potassium | ▪ | Reduced risk of high blood pressure and stroke |
| ▪ | Diets low in sodium | | |
| ▪ | Plant sterol and stanol esters | ▪ | Reduced risk of coronary heart disease |
| ▪ | Soy protein | | |
| ▪ | Fruits, vegetables, and grain products that contain fiber, particularly soluble fiber | | |
| ▪ | Diets low in dietary saturated fat and cholesterol | | |
| ▪ | Calcium | ▪ | Reduced risk of osteoporosis |
| ▪ | Fruits, vegetables, and liber-containing grain products | ▪ | Reduced risk of cancer |
| ▪ | Diets low in dietary fat | | |
| ▪ | Folate | ▪ | Reduced risk of neural tube birth defects |
| ▪ | Dietary sugar alcohol | ▪ | Reduced risk of dental caries (cavities) |

Although not yet approved by the FDA for the purposes of labeling, numerous other functional foods are believed to provide health benefits beyond those listed above, such as reduced inflammation.

Functional ingredients generally are classified into categories such as carotenoids, dietary fiber, fatty acids, flavonoids, isothiocyanates, phenols, plant sterols and stanols (phytosterols and phytostanols); polyols; prebiotics/probiotics; phytoestrogens; soy protein; sulfides/thiols; amino acids; proteins; vitamins; and minerals. Functional ingredients also may be classified based on their health benefits, such as cardiovascular, cholesterol-reducing, and anti-inflammatory.

Health trends also have promoted an increased use of non-caloric high-potency sweeteners in consumer diets. Although natural caloric sweetener compositions, such as sucrose, fructose, and glucose, provide the most desirable taste to consumers, they are caloric. Numerous natural and synthetic high-potency sweeteners are non-caloric; however, they exhibit sweet tastes that have different temporal profles, maximal responses, flavor profiles, mouthfeels, and/or adaptation behaviors than that of sugar.

For example, the sweet tastes of natural and synthetic high-potency sweeteners are slower in onset and longer in duration than the sweet taste produced by sugar and thus change the taste balance of a food composition. Because of these differences, use of natural and synthetic high-potency sweeteners to replace a bulk sweetener, such as sugar, in a food or beverage, causes an unbalanced temporal profile and/or flavor profile. In addition to the difference in temporal profile, high-potency sweeteners generally exhibit (i) lower maximal response than sugar, (ii) off tastes including bitter, metallic, cooling, astringent, licorice-like taste, etc., and/or (iii) sweetness which diminishes on iterative tasting. It is well known to those skilled in the art of food/beverage formulation that changing the sweetener in a composition requires re-balancing of the flavor and other taste components (e.g., acidulants). If the taste profile of natural and synthetic high-potency sweeteners could be modified to impart specific desired taste characteristics to be more sugar-like, the type and variety of compositions that may be prepared with that sweetener would be expanded significantly. Accordingly, it would be desirable to selectively modify the taste characteristics of natural and synthetic high-potency sweeteners.

JP 2003-180288 A discloses an oral material with improved taste, wherein sweet characteristics, especially the bitterness, the aftertaste and the sweet feeling can be improved by combi-nation of an intense sweetener, polyol, an enzyme-treated gingko biloba compound. A stevia sweetener is mentioned as a suitable intense sweetener.

JP 2004-073197 A discloses a sweetening composition suitable to provide desirable taste characteristics to orally taken products. Provided is a combination of an intense sweetener such as stevioside, a polyol, and arabinose.

JP 2000-236842 A provides a stevia sweetener with improved bitterness and sweet aftertaste. Specifically, the taste properties of α-glycosilated steviol glycoside sweeteners can be improved by purifying the stevia-extract starting material to contain a high rebaudioside A concentration followed by enzymatic modification to produce functionalized α-glycosilated steviol glycosides, which are then combined with a polyol such as erythritol to produce a more complex synthetic sweetener composition.

It also would be desirable to improve the taste of ingestible compositions that include functional ingredients to promote their use and the resulting health benefits.

### SUMMARY OF THE INVENTION

Generally, this invention addresses the above described need by providing a functional sweetener composition having improved temporal profile and/or flavor profile, a method for improving the temporal profile and/or flavor profile, a functional sweetener composition having improved temporal and/or flavor profile, and a method for improving the temporal profile and/or flavor profile for a functional sweetener composition. In particular, this invention improves the temporal profile and/or flavor profile by imparting a more sugar-like temporal profle and/or flavor profile. More particularly, this invention comprises a functional sweetener composition according to claim 1.

Objects and advantages of the invention will be set forth in part in the following description, or may be obvious from the description, or may be learned through practice of the invention. Unless otherwise defined, all technical and scientific terms and abbreviations used herein have the same meaning as commonly understood by one of ordinary skill in the art to which this invention pertains. Although methods and compositions similar or equivalent to those described herein can be used in practice of the present invention, suitable methods and compositions are described without intending that any such methods and compositions limit the invention herein.
According to one aspect of the invention a functional sweetener composition is provided according to claim 1.
In one preferred embodiment of the functional sweetener composition the at least one agent for the treatment and/or prevention of autoimmune disorders comprises at least one probiotic, at least one prebiotic, at least one fatty acid, at least one antioxidant, or combinations thereof.
In one preferred embodiment of the functional sweetener composition the at least one probiotic comprises a bacteria selected from the genus *Bacillus*, *Lactobacillus*, *Bifodobaclerium*, and combinations thereof.
In one preferred embodiment of the functional sweetener composition the at least one prebiotic comprises fructooligosaccharides, inulins, isomalto-oligosaccharides, lactilols, lactosucroses, lactuloses, pyrodextrins, soy oligosaccharides, transgalacto-oligosaccharides, xylo-oligosaccharides, or combinations thereof.
In one preferred embodiment of the functional sweetener composition the at least one fatty acid comprises an omega-3-fatty acid.
In one preferred embodiment of the functional sweetener composition the at least one antioxidant comprises vitamin cofactors, minerals, hormones, carotenoids, carotenoid terpenoids, non-carotenoid terpenoids, flavonoid polyphenolics, flavonols, flavones, phenols, polyphenols, esters of phenols, esters of polyphenols, nonflavonoid phenolics, isothiocyanates, or combinations thereof.

In the functional sweetener composition erythritol is a first sweet taste improving composition.

In the functional sweetener composition the erythritol imparts a more sugar-like temporal profile to the functional sweetener composition than the rebaudioside A would have without the erythritol.

One preferred embodiment of the functional sweetener composition further comprises at least one second sweet taste improving composition different from erythritol and selected from the group consisting of carbohydrates, polyols, amino acids and their corresponding salts, polyamino acids and their corresponding salts, sugar acids and their corresponding salts, organic acids, inorganic acids, organic salts, inorganic salts, bitter compounds, flavorants, astringent compounds, polymers, protein hydrolysates, surfactants, emulsifiers, flavanoids, alcohols, and combinations thereof.

One preferred embodiment of the functional sweetener composition further comprises at least one third sweet taste improving composition different from erythritol and the at least one second sweet taste improving composition and selected from the group consisting of carbohydrates, polyols, amino acids and their corresponding salts, polyamino acids and their corresponding salts, sugar acids and their corresponding salts, organic acids, inorganic acids, organic salts, inorganic salts, bitter compounds, flavorants, astringent compounds, polymers, protein hydrolysates, surfactants, emulsifiers, flavanoids, alcohols, and combinations thereof.

In one preferred embodiment of the functional sweetener composition the amino acid comprises glycine, alanine, proline, hydroxyproline, glutamine, or combinations thereof.

In one preferred embodiment of the functional sweetener composition the polyamino acid comprises poly-L-aspartic acid, poly-L-α-lysine, poly-L-ε-lysine, poly-L-α-ornithine, poly-ε-ornithine, poly-L-arginine, salts thereof, or combinations thereof.

In one preferred embodiment of the frictional sweetener composition the inorganic salt comprises a sodium, potassium, calcium, or magnesium salt.

One preferred embodiment of the functional sweetener composition further comprises at least one inorganic phosphate.

In one preferred embodiment of the functional sweetener composition the at least one inorganic phosphate comprises a sodium, potassium, calcium, or magnesium phosphate.

One preferred embodiment of the functional sweetener composition further comprises at least one inorganic chloride.

In one preferred embodiment of the functional sweetener composition the at least one inorganic chloride comprises a sodium, potassium, calcium, or magnesium chloride.

In one preferred embodiment of the functional sweetener composition the carbohydrate comprises sucrose, high fructose corn syrup, glucose, or sucrose.

In one preferred embodiment of the functional sweetener composition the carbohydrate is present in the functional sweetener composition in an amount from about 10,000 ppm to about 80,000 ppm of the composition.

In one preferred embodiment of the functional sweetener composition the at least one sweet taste improving composition comprises at least one synthetic high-potency sweetener.

In one preferred embodiment of the functional sweetener composition the at least one synthetic high-potency sweetener comprises sucralose, acesulfame potassium or other salts, aspartame, alitame, saccharin, neohesperidin dihydrochalcone, cyclamate, neotame, N-[N-[3-(3-hydroxy-4-methoxyphenyl)propyl]-L-α-aspartyl]-L-phenylalanine 1-methyl ester, N-[N-[3-(3-hydroxy-4-methoxyphenyl)-3-methylbutyl]-L-α-aspartyl]-L-phenylalanine 1-methyl ester, N-[N-[3-(3-methoxy-4-hydroxyphenyl)propyl]-L-α-aspartyl]-L-phenylalanine 1-methyl ester, salts thereof, and combinations thereof.

In one preferred embodiment of the functional sweetener composition the at least one synthetic high-potency sweetener comprises saccharin or acesulfame potassium or other salts.

In one preferred embodiment of the functional sweetener composition the at least one synthetic sweetener is present in the functional sweetener composition in an amount from about 10 ppm to about 100 ppm of the composition.

In one preferred embodiment of the functional sweetener composition the ) rebaudioside A comprises rebaudioside A in a purity greater than about 70 % rebaudioside A by weight on a dry basis.

In one preferred embodiment of the functional sweetener composition the rebaudioside A comprises rebaudioside A in a purity greater than about 80 % rebaudioside A by weight on a dry basis.

In one preferred embodiment of the functional sweetener composition the rebaudioside A comprises rebaudioside A in a purity greater than about 90 % rebaudioside A by weight on a dry basis.

In one preferred embodiment of the functional sweetener composition the rebaudioside A comprises rebaudioside A in a purity greater than about 97 % rebaudioside A by weight on a dry basis.

In one preferred embodiment of the functional sweetener composition the rebaudioside A comprises rebaudioside A in a purity greater than about 98 % rebaudioside A by weight on a dry basis.

In one preferred embodiment of the functional sweetener composition the rebaudioside A comprises rebaudioside A in a purity greater than about 99 % rebaudioside A by weight on a dry basis.

According to another aspect of the invention a functional sweetened composition is provided according to claim 2.
In one preferred embodiment of the functional sweetened composition the at least one agent for the treatment and/or prevention of autoimmune disorders comprises at least one probiotic, at least one prebiotic, at least one fatty acid, at least one antioxidant, or combinations thereof.

In one preferred embodiment of the functional sweetened composition the at least one probiotic comprises a bacteria selected from the genus *Bacillus, Lactobacillu, Bifodobacterium,* and combinations thereof.
In one preferred embodiment of the functional sweetened composition the at least one prebiotic comprises fructooligosaccharides, inulins, isomalto-oligosaccharides, lactilols, lactosucroses, lactuloses, pyrodextrins, soy oligosaccharides, transgalacto-oligosaccharides, xylo-oligosaccharides, or combinations thereof.
In one preferred embodiment of the functional sweetened composition the at least one fatty acid comprises an omega-3-fatty acid.

In one preferred embodiment of the functional sweetened composition the at least one antioxidant comprises vitamin cofactors, minerals, hormones, carotenoids, carotenoid terpenoids, non-carotenoid terpenoids, flavonoid polyphenolics, flavonols, flavones, phenols, polyphenols, esters of phenols, esters of polyphenols, nonflavonoid phenolics, isothiocyanates, or combinations thereof.

In the functional sweetened composition erythritol is a first sweet taste improving composition.

In the functional sweetened composition the erythritol imparts a more sugar-like temporal profile to the sweetened composition than the rebaudioside A would have without the erythritol.

One preferred embodiment of the functional sweetened composition further comprises at least one second sweet taste improving composition different from the erythritol first sweet taste improving composition and selected from the group consisting of carbohydrates, polyols, amino acids and their corresponding salts, polyamino acids and their corresponding salts, sugar acids and their corresponding salts, organic acids, inorganic acids, organic salts, inorganic salts, bitter compounds, flavorants, astringent compounds, polymers, protein hydrolysates, surfactants, emulsifiers, flavonoids, alcohols, chelating agents, and combinations thereof.

One preferred embodiment of the functional sweetened composition further comprises at least one third sweet taste improving composition different from the erythritol and the at least one second sweet taste improving composition and selected from the group consisting of carbohydrates, polyols, amino acids and their corresponding salts, polyamino acids and their corresponding salts, sugar acids and their corresponding salts, organic acids, inorganic acids, organic salts, inorganic salts, bitter compounds, flavorants, astringent compounds, polymers, protein hydrolysates, surfactants, emulsifiers, flavonoids, alcohols, chelating agents, and combinations thereof.

In one preferred embodiment of the functional sweetened composition the amino acid comprises glycine, alanine, proline, hydroxyproline, glutamine, or combinations thereof.

In one preferred embodiment of the functional sweetened composition the at least one sweet taste improving composition comprises at least one polyamino acid.

In one preferred embodiment of the functional sweetened composition the polyamino acid comprises poly-L-aspartic acid, poly-L-lysine, poly-L-ornithine, poly-ε-ornithine, poly-L-arginine, salts thereof, or combinations thereof.

In one preferred embodiment of the functional sweetened composition the inorganic salt comprises a sodium, potassium, calcium, or magnesium salt.

One preferred embodiment of the functional sweetened composition further comprises at least one inorganic phosphate.

In one preferred embodiment of the functional sweetened composition the at least one inorganic phosphate comprises a sodium, potassium, calcium, or magnesium phosphate.

One preferred embodiment of the functional sweetened composition further comprises at least one inorganic chloride.

In one preferred embodiment of the functional sweetened composition the at least one inorganic chloride comprises a sodium, potassium, calcium, or magnesium chloride.

In one preferred embodiment of the functional sweetened composition the carbohydrate comprises sucrose, high fructose corn syrup, glucose, or sucrose.

In one preferred embodiment of the functional sweetened composition the carbohydrate is present in the functional sweetened composition in an amount from about 10,000 ppm to about 80,000 ppm of the composition.

In one preferred embodiment of the functional sweetened composition the at least one sweet taste improving composition comprises at least one synthetic high-potency sweetener.

In one preferred embodiment of the functional sweetened composition the at least one synthetic high-potency sweetener comprises sucralose, acesulfame potassium or other salts, aspartame, alitame, saccharin, neohesperidin dihydrochalcone, cyclamate, neotame, N-[N-[3-(3-hydroxy-4-methoxyphenyl)propyl]-L-α-aspartyl]-L-phenylalanine 1-methyl ester, N-[N-[3-(3-hydroxy-4-methoxyphenyl)-3-methylbutyl]-L-α-asparty1]-L-phenylalanine 1-methyl ester, N-[N-[3-(3-methoxy-4-hydroxyphenyl)propyl]-L-α-aspartyl]-L-phenylalanine 1-methyl ester, salts thereof, and combinations thereof.

In one preferred embodiment of the functional sweetened composition the at least one synthetic high-potency sweetener comprises saccharin or acesulfame potassium or other salts.

In one preferred embodiment of the functional sweetened composition the at least one synthetic sweetener is present in the functional sweetened composition in an amount from about 10 ppm to about 100 ppm of the composition.

In one preferred embodiment of the functional sweetened composition the rebaudioside A comprises rebaudioside A in a purity greater than about 70 % rebaudioside A by weight on a dry basis.

In one preferred embodiment of the functional sweetened composition the rebaudioside A comprises rebaudioside A in a purity greater than about 80 % rebaudioside A by weight on a dry basis.

In one preferred embodiment of the functional sweetened composition the rebaudioside A comprises rebaudioside A in a purity greater than about 90 % rebaudioside A by weight on a dry basis.

In one preferred embodiment of the functional sweetened composition the rebaudioside A comprises rebaudioside A in a purity greater than about 97 % rebaudioside A by weight on a dry basis.

In one preferred embodiment of the functional sweetened composition the rebaudioside A comprises rebaudioside A in a purity greater than about 98 % rebaudioside A by weight on a dry basis.

In one preferred embodiment of the functional sweetened composition the rebaudioside A comprises rebaudioside A in a purity greater than about 99 % rebaudioside A by weight on a dry basis.

In one preferred embodiment of the functional sweetened composition the sweetened composition comprises a food, beverage, pharmaceutical, tobacco, nutraceutical, oral hygienic, or cosmetic.

According to another aspect of the invention a functional beverage according to claim 7 is provided, comprising the sweetened composition.

In one preferred embodiment of the functional beverage the beverage is a non-carbonated beverage or a carbonated beverage.

In one preferred embodiment of the functional beverage the carbonated beverage is a cola.

In one preferred embodiment of the functional beverage the carbonated beverage is a fruit-flavored beverage.

In one preferred embodiment of the functional beverage the carbonated beverage is a citrus-flavored beverage.

In one preferred embodiment of the functional beverage the citrus-flavored beverage is a lemon-lime flavored beverage or a orange-flavored beverage.

In one preferred embodiment of the functional beverage the carbonated beverage is a root beer.

In one preferred embodiment of the functional beverage the beverage is a fruit juice, fruit-flavored, or fruit-containing beverage.

In one preferred embodiment of the functional beverage the beverage is a vegetable juice or vegetable containing beverage.

In one preferred embodiment of the functional beverage the beverage is tea.

In one preferred embodiment of the functional beverage the beverage is coffee.

In one preferred embodiment of the functional beverage the beverage comprises a dairy component.

In one preferred embodiment of the functional beverage the beverage is a sports drink.

In one preferred embodiment of the functional beverage the beverage is an energy drink.

In one preferred embodiment of the functional beverage the beverage is a flavored water.

### BRIEF DESCRIPTION OF THE DRAWINGS

Fig. 1 is a powder x-ray diffraction scan of rebaudioside A polymorph Form I on a plot of the scattering intensity versus the scattering angle 2θ in accordance with an embodiment of this invention.
Fig. 2 is a powder x-ray diffraction scan of rebaudioside A polymorph Form 2 on a plot of the scattering intensity versus the scattering angle 2θ in accordance with an embodiment of this invention.
Fig. 3 is a powder x-ray diffraction scan of rebaudioside A polymorph Form 3A on a plot of the scattering intensity versus the scattering angle 2θ in accordance with an embodiment of this invention.
Fig. 4 is a powder x-ray diffraction scan of rebaudioside A polymorph Form 3B on a plot of the scattering intensity versus the scattering angle 2θ in accordance with an embodiment of this invention.
Fig. 5 is a powder x-ray diffraction scan of rebaudioside A polymorph Form 4 on a plot of the scattering intensity versus the scattering angle 2θ in accordance with an embodiment of this invention.

### DETAILED DESCRIPTION OF THE INVENTION

Reference now will be made in detail to the presently proffered embodiments of the invention. Each example is provided by way of explanation of embodiments of the invention, not limitation of the invention. In fact, it will be apparent to those skilled in the art that various modifications and variations can be made in the present invention without departing from the spirit or scope of the invention. For instance, features illustrated or described as part of one embodiment, can be used on another embodiment to yield a still further embodiment. Thus, it is intended that the present invention cover such modifications and variations within the scope of the appended claims and their equivalents.

Embodiments of this invention include functional sweetener compositions and functional sweetened compositions. Also embodied in this invention are methods for making functional sweetener compositions and functional sweetened compositions.

### I. Functional Ingredients

The functional ingredient comprises an agent for the treatment and/or prevention of autoimmune disorders.

Autoimmune disorders occur when the immune system mounts an immune response against normal body tissues. It is estimated that 5 to 8 % of persons in the United States (14 to 22 million persons) are affected by autoimmune disorders. Most autoimmune disorders disproportionately affect women and are among the 10 leading causes of death for women in every age group up to 64 years of age. However, autoimmune disorders also affect persons of all racial and ethnic groups with onset from childhood to late adulthood. The exact cause of autoimmune disorders is unknown; however, there appears to be both an inherited predisposition to the development of several types of autoimmune diseases as well as triggers, such as microorganisms, to the development of autoimmune diseases.

More than 80 clinically distinct autoimmune diseases have been identified. Autoimmune diseases generally are divided into two groups: organ-specific and systemic. Organ-specific autoimmune diseases are localized in one organ. Systemic autoimmune diseases, also referred to as collagen vascular diseases, are expressed widely in the body and often involve multiple organs.

Common types of autoimmune disorders and the tissues involved include.:

| **Tissue** | **Autoimmune disease (S = Systemic)** |
|---|---|
| Blood or vasculature | • Autoimmune hemolytic anemia |
| | • Pernicious anemia |
| | • Polyarteritis nodosa |
| | • Systemic lupus erythematosus (S) |
| | • Wegener's granulomatosis (S) |
| Gastraintestinal system | • Autoimmune hepatitis |
| | • Behget's disease |
| | • Crohn's disease |
| | • Primary biliary cirrhosis |
| | • Seleroderma (S) |
| | • Ulcerative colitis |
| | • Irritible Bowel Syndrome |
| Ocular System | • Sjögren's syndrome (S) |
| | • Type 1 diabetes mellitus |
| | • Uveitis |
| Endocrine System | • Graves' disease |
| | • Thyroiditis |
| Cardiovascular system | • Myocarditis |
| | • Rheumatic fever |
| | • Systemic lupus erythematosus (S) |
| Connective tissue | • Ankylosing spondylitis (S) |
| | • Rheumatoid and reactive arthritis (S) |
| | • Systemic lupus erythematosus (S) |
| Kidneys | • Glomerulonephritis |
| | • Goodpasture's syndrome (S) |
| Lungs | • Sarcoidosis |
| | • Goodpasture's syndrome (S) |
| Musculoskeletal system | • Dermatomyositis |
| | • Myasthenia gravis |
| | • Polymyositis |
| | • Fibromialgia |
| Neurological system | • Guilliain-Barre syndrome |
| | • Multiple sclerosis |
| Skin | • Alopecia areata |
| | • Pemphigus |
| | • Psoriasis |
| | • Vitiligo |

It would be desirable to supplement orally ingestible products that are consumed daily with agents for the treatment and/or prevention of autoimmune disorders. The functional sweetener composition or functional sweetened composition comprises at least one functional ingredient, the at least one functional ingredient comprising an agent for the treatment and/or prevention of autoimmtune disorders. In a particular embodiment, the at least one agent for the treatment and/or prevention of autoimmune disorders comprises a probiotic, a prebiotic, a fatty acid, an antioxidant, or combinations thereof.

Probiotics are believed to be effective at modulating the immune response; however, the mechanisms by which probiotics effect immunomodulation are unclear. Probiotic modulation of the immune system may be through modulation of humoral responses, non-specific immunity, or combinations thereof. For example, it is well known to those of ordinary skill in the art that excessive activation of immune effector cells and overproduction of certain cytokines can cause severe inflammation and tissue damage. Many species of bacteria, yeasts, and fungi may be used in the prophylactic or therapeutic treatment of autoimmune disorders by producing and excreting enzymes useful in digestion (e.g., lactase, proteases, lipases, and amylases), by down-regulating cytokine responses of mucosal cells, and by maintaining a healthy gastrointestinal tract (e.g., containing growth of harmful microorganisms.

In a particular embodiment, the probiotic comprises a microorganism selected from the group consisting of bacteria, yeast, fungi, and combinations thereof. According to a particularly desirable embodiment of the invention, the probiotic comprises a bacteria selected from the genus *Bacillus, Lactobacillus, Bifodobacterium,* and combinations thereof. Non-limiting examples of species of bacteria in the *Bacillus* genus include *B. coagulans.* Non-limiting examples of species of bacteria in the *Lactobacilllus* genus include *L. acidophilus, L. casei, L. fermentum, L. saliva roes, L. brevis, L. leichmannii, L. plantarum, L. cellobiosus, L. reuteri, L. rhamnosus, L. GG, L. bulgaricus, L. thermophilus, L. paracasei,* and *L. buchneri.* Non-limiting examples of species of bacteria in the *Bifodobacterium* genus include *B. adolescentis, B. angulatum, B. animalis, B. asteroides, B. bifidum, B. boum, B. breve, B. catenulatum, B. choerinum, B. coryneforme, B. cuniculi, B. dentium, B. gallicum, B. gallinarum, B indicum, B. infantis, B. longum, B. magnum, B. merycicum, B. minimum, B. pseudocatenailatum, B. pseudolongum, B. psychraerophilum, B. pullorum, B. ruminantium, B. saeculare, B. scardovii, B. simiae, B. subtile, B. thermacidophilum. B. thermophilum, B. urinalis, and B. sp.* The bacteria provided herein may be in any form known to those of ordinary skill in the art, non-limiting examples of which include vegetative cells and spores. The probiotics provided herein also may comprise a delivery system for the protection of the probiotics from stomach acid, non-limiting examples of which include enteric coated capsules and microencapsulation.

In another particular embodiment of the invention, the at least one agent for the treatment and/or prevention of autoimmune disorders comprises a prebiotic. Prebiotics, as used herein, comprise compositions that promote the growth of beneficial bacteria in the intestines. Non-limiting examples of prebiotics include mucopolysaccharides, oligosaccharides, polysaccharides, amino acids, vitamins, nutrient precursors, and proteins. Particular embodiments of functional sweetener compositions comprising prebiotics are discussed in more detail in the co-pending U.S. Patent Application No. 11/556,916, entitled "High-Potency Sweetener Composition with Probiotics/Prebiotics and Compositions Sweetened Therewith," filed on November 2, 2006, by Indra Prakash and Grant Dubois.

Prebiotic substances generally are consumed by a relevant probiotic, or otherwise assist in keeping the relevant probiotic alive or stimulate its growth. When consumed in an effective amount, prebiotics also benefit the naturally-occurring gastrointestinal microflora. A healthy gastrointestinal tract with adequate mucus production and bacterial colonigzation can prevent or inhibit the growth of pathogenic or opportunistic microorganisms, thereby modulating diseases processes and preventing widespread inflammatory disorders.

According to a particular embodiment of this invention, the at least one prebiotic comprises dietary fibers, including, without limitation, polysaccharides and oligosaccharides. Non-limiting examples of oligosaccharides that are categorized as prebiotics in accordance with particular embodiments of this invention include fructooligosaccharides, inulins, isomalto-oligosaccharides, lactilol, lactosucrose, lactulose, pyrodextrins, soy oligosaccharides, transgalacto-oligosaccharides, and xylo-oligosaccharides.

In yet another particular embodiment of the invention, the at least one agent for the treatment and/or prevention of autoimmune disorders comprises a fatty acid. Particular embodiments of functional sweetener compositions comprising fatty acids are discussed in more detail in the co-pending U.S. Patent Application No. 11/555,766, entitled "High-Potency Sweetener Composition with Polyunstaurated Fatty Acid and Compositions Sweetened Therewith," filed on November 2, 2006, by DuBois, et al., the disclosure of which is incorporated herein by reference in its entirety.

Not wishing to be bound by any theory, it is believed that competition occurs between the omega-6 and omega-3 fatty acids in prostaglandin formation. It also is believed that omega-3 suppresses the capacity of monocytes to synthesize inflammatory and autoimmune cytokines (e.g., IL-1 and TNF). Accordingly, in particularly desirable embodiments of the present invention, the fatty acid comprises an omega-3 fatty acid. Non-limiting examples of omega-3 fatty acids include eicosapentaeonic acid and docosahexaenoic acid. Wherein the at least one agent for the treatment and/or prevention of autoimmune disorders comprises a fatty acid, the fatty acid desirably is present in the functional sweetener composition in an amount in the range of about 0.002 percent by weight to about 35 percent by weight of the functional sweetener composition. In another particular embodiment, the fatty acid desirably is present in a functional sweetened composition in an amount in the range of about 25 to about 35 mg per serving (~240 mL).

In still another particular embodiment of the invention, the at least one agent for the treatment and/or prevention of autoimmune disorders comprises an antioxidant. Particular embodiments of functional sweetener compositions comprising antioxidant are discussed in more detail in the co-pending U.S. Patent Application No. 11/556,095, entitled "High-Potency Sweetener Composition with Antioxidant and Compositions Sweetened Therewith," filed on November 2, 2006, by Indra Prakash and Grant DuBois, the disclosure of which is incorporated herein by reference in its entirety.

According to particular embodiments of this invention, non-limiting examples of antioxidants include vitamin cofactors, minerals, hormones, carotenoids, carotenoid terpenoids, non-carotenoid terpenoids, flavonoid polyphenolics, flavonols, flavones, phenols, polyphenols, esters of phenol, esters of polyphenols, nonflavonoid phenolics, isothiocyanates, and combinations thereof.

In particular embodiments of the present invention, the antioxidant comprises polyphenols. Polyphenols generally are found in plants and are characterized by the presence of more than one phenol group per molecule. Suitable polyphenols for embodiments of this invention include, but are not limited to, catechins, proanthocyanidins, procyanidins, other similar materials, and combinations thereof.

In particular embodiments, the agent for the treatment and/or prevention of autoimmune disorders comprises a catechin. Suitable catechins include, but are not limited to, epigallocatechin gallate (EGCG), catechin gallate (CG), epicatechin (EC), epigallocatechin (EGC), and gallocatechin gallate (GCG). Catechins may be obtained from numerous sources, non-limiting examples of which include green tea or green tea extract, other teas or tea extracts (e.g., white tea, black tea, or oolong tea), chocolate/coca, red wine, grape seed extract, grape skin or grape juice (from red or purple grapes), benies, red apple peel, or the like. In addition, catechins may be extracted from Pycnogenol from French maritime pine bark extract. According to particular embodiments of the present invention, EGCG is present in the in the functional sweetener composition in an amount from about 90 mg to about 270 mg per serving (~ 240 mL). In other embodiments, green tea extract is present in the in the functional sweetener composition in an amount from about 500 mg to about 600 mg per serving (~ 240 mL).

In another particular embodiment, the agent for the treatment and/or prevention of autoimmune disorders comprises proanthocyanidins, procyanidins, or combinations thereof. Suitable sources of proanthocyanidins and procyanidins for embodiments of this invention include, but are not limited to, grape seed extracts, cacao/cocoa/chocolate extracts, grape skin or grape juice (from red or purple grapes), apple peel, colorful berries, sorghum, cinnamon, barley, and hops. In addition, proanthocyanidins and procyanidins may be obtained from Pycnogenol. According to particular embodiments of the present invention, grape seed extract is present in the functional sweetener composition in an amount from about 100 mg to about 200 mg per serving. In other embodiments, cocoa extract is present in the functional sweetener composition in an amount from about 400 mg to 600 mg about per serving. In still another embodiment, Pycogenol is present in the functional sweetener composition in an amount from about 75 to about 200 mg per serving.

In some embodiments, the antioxidant may include ubiquinone, mineral selenium, manganese, melatonin, α-carotene, β-carotene, lycopene, lutein, zeanthin, crypoxanthin, reservatol, eugenol, quercetin, catechin, gossypol, hesperetin, curcumin, ferulic acid, thymol, hydroxytyrosol, tumeric, thyme, olive oil, lipoic acid, glutathinone, gutamine, oxalic acid, tocopherol-derived compounds, butylated hydroxyanisole (BHA), butylated hydroxytoluene (BHT), ethylenediaminetetraacetic acid (EDTA), tert-butylhydroquinone, acetic acid, pectin, tocotrienol, tocopherol, coenzyme Q10, zeaxanthin, astaxanttun, canthaxantin, saponins, limonoids, kaempfedrol, myricetin, isorhamnetin, proanthocyanidins, quercetin, rutin, luteolin, apigenin, tangeritin, hesperetin, naringenin, erodictyol, flavan-3-ols (e.g., anthocyanidins), gallocatechins, epicatechin and its gallate forms, epigallocatechin and its gallate formes (ECGC) theaflavin and its gallate forms, thearubigins, isoflavone phytoestrogens, genistein, daidzein, glycitein, anythucyanins, cyaniding, delphinidin, malvidin, pelargonidin, peonidin, petunidin, ellagic acid, gallic acid, salicylic acid, rosmarinic acid, cinnamic acid and its derivatives (e.g., ferulic acid), chlorogenic acid, chicoric acid, gallotannins, ellagitannins, anthoxanthins, betacyanins and other plant pigments, silymarin, citric acid, lignan, antinutrients, bilirubin, uric acid, R-α-iipoic acid, N-acetylcysteine, emblicanin, and phytic acid, or combinations thereof.

In alternate embodiments, the antioxidant may comprise a synthetic antioxidant such as butylated hydroxytolune or butylated hydroxyanisole, for example. Other sources of suitable antioxidants for embodiments of this invention include, but are not limited to, fruits, vegetables, tea, cocoa, chocolate, spices, herbs, rice, organ meats from livestock, yeast, whole grains, or cereal grains.

Generally, the amount of antioxidant present in the functional sweetener composition varies widely depending on the particular functional sweetener composition and the desired antioxidant. Those of ordinary skill in the art will readily acertain the appropriate amount of antioxidant for each functional sweetener composition. In embodiments, the functional sweetened composition may comprise up to 25 ppm to 35 ppm of polyphenol per 240 mL serving. In other embodiments, the functional sweetened composition may comprise up to 5 mg to 15 mg of coenzyme Q10 per 240 mL serving.

It is well known to those of ordinary skill in the art that phytonutrients, plant extracts, and herbal compositions may be used in their natural and/or modified form. Modified phytonutrient, plant extracts, and herbal compositions include phytonutrients, plant extracts, and herbal compositions which have been altered naturally. For example, a modified phytonutrient includes, but is not limited to, phytonutrients which have been fermented, contacted with enzyme, or derivatized or substituted on the phytonutrient. In one embodiment, modified phytonutrients may be used individually or in combination with unmodified phytonutrients. For the sake of brevity, however, in the description of embodiments of this invention, a modified phytonutrient is not described expressly as an alternative to an unmodified phytonutrient, but it should be understood that modified phytonutrients can be substituted for or combined with phytonutrients in any embodiment disclosed herein. The same embodiments would be applicable to plant extracts and other herbal compositions. Plant extracts include extracts from foliage, stems, bark, fruit, seed, and any other plant matter.

The amount of the at least one agent for the treatment and/or prevention of autoimmune disorders present in the functional sweetener composition or functional sweetened composition will vary depending on the individual agent for the treatment and/or prevention of autoimmune disorders or combination of agents for the treatment and/or prevention of autoimmune disorders. Those of ordinary skill in the art can readily discern the appropriate amount of an agent for the treatment and/or prevention of autoimmune disorders to put in the functional sweetener composition or functional sweetened composition. In a particular embodiment, the at least one agent for the treatment and/or prevention of autoimmune disorders is present in the functional sweetener composition or functional sweetened composition in an amount sufficient to promote health and wellness.

According to particular embodiments of this invention, the sweetener compositions provided herein further may comprise at least one functional ingredient different than the at least one agent for the treatment and/or prevention of autoimmune disorders described hereinabove. According to particular embodiments of this invention, non-limiting examples of such functional ingredients include naturally nutrient-rich or medicinally active food, such as garlic, soybeans, antioxidants, fibers, glucosamine, chondroitin sulfate, ginseng, ginko, Echinacea, or the like; other nutrients that provide health benefits, such as amino acids, vitamins, minerals, carotenoids, dietary fiber, fatty acids such as omega-3 or omega-6 fatty acids, DHA, EPA, or ALA which can be derived from plant or animal sources (e.g., salmon and other cold-water fish or algae), flavonoids, phenols, polyols, polyphenols (e.g., catechins, proanthocyanidins, procyanidins, anthocyanins, quercetin, resveratrol, isoflavones, curcumin, punicalagin, ellagitannin, citrus flavonoids such as hesperidin and naringin, and chlorogenic acid), prebiotics/probiotics, phytoestrogens, sulfides/thiols, policosanol, saponin, rubisco peptide, appetite suppressants, hydration agents, C-reactive protein reducing agents, or anti-inflammatory agents; or any other functional ingredient that is beneficial to the treatment of specific diseases or conditions, such as diabetes, osteoporosis, inflammation, or cholesterol.

### II. Natural and/or Synthetic High-Potency Sweeteners

The sweetener compositions provided also comprise rebaudioside A. As used herein the phrases "natural high-potency sweetener", "NHPS", "NHPS composition", and "natural high-potency sweetener composition" are synonymous. "NHPS" means any sweetener found in nature which may be in raw, extracted, purified, or any other form, singularly or in combination thereof and characteristically have a sweetness potency greater than sucrose, fructose, or glucose, yet have less calories. Non-limiting examples of NHPSs include rebaudioside A, rebaudioside B, rebaudioside C, rebaudioside D, rebaudioside E, rebaudioside F, dulcoside A, dulcoside B, rubusoside, stevia, stevioside, mogroside IV, mogroside V, Luo Han Guo sweetener, siamenoside, monatin and its salts (monatin SS, RR, RS, SR), curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, and cyclocarioside 1. NHPS also includes modified NHPSs. Modified NHPSs include NHPSs which have been altered naturally. For example, a modified NHPS includes, but is not limited to, NHPSs which have been fennented, contacted with enzyme, or derivatized or substituted on the NHPS. At least one modified NHPS may be used in combination with at least one NHPS. At least one modified NHPS may be used without a NHPS. Thus, modified NHPSs may be substituted for a NHPS or may be used in combination with NHPSs. For the sake of brevity, however, in the description a modified NHPS is not expressly described as an alternative to an unmodified NHPS, but it should be understood that modified NHPSs can be substituted for NHPSs.

Extracts of a NHPS may be used in any purity percentage. When a NHPS is used as a non-extract, the purity of the NHPS may range for example from about 25% to about 100%. The purity of the NHPS may range from about 50% to about 100%; from about 70% to about 100%; from about 80% to about 100%; from about 90% to about 100%; from about 95% to about 100%; from about 95% to about 99.5%; from about 96% to about 100%; from about 97% to about 100%; from about 98% to about 100%; and from about 99% to about 100%.

Purity, as used here, represents the weight percentage of a respective NHPS compound present in a NHPS extract, in raw or purified form. A steviolglycoside extract may comprise a particular steviolglycoside in a particular purity, with the remainder of the stevioglycoside extract comprising a mixture of other steviolglycosides.

To obtain a particularly pure extract of a NHPS, such as rebaudioside A, it may be necessary to purify the crude extract to a substantially pure form. Such methods generally are known to those of ordinary skill in the art.

An exemplary method for purifying a NHPS, such as rebaudioside A, is described in the co-pending patent application no. 60/805,216, entitled "Rebaudioside A Composition and Method for Purifying Rebaudioside A," filed on June 19, 2006, by inventors DuBois, et al..

Briefly described, substantially pure rebaudioside A is crystallized in a single step from an aqueous organic solution comprising at least one organic solvent and water in an amount from about 10 % to about 25 % by weight, more particularly from about 15 % to about 20 % by weight. Organic solvents desirably comprise alcohols, acetone, and acetonitile. Non-limiting examples of alcohols include ethanol, methanol, isopranol, 1-propanol, 1-bulanol, 2-butanol, tert-butanol, and isobutanol. Desirably, the at least one organic solvent comprises a mixture of ethanol and methanol present in the aqueous organic solution in a weight ratio ranging from about 20 parts to about 1 part ethanol to I part methanol, more desirably from about 3 parts to about 1 part ethanol to 1 part methanol.

Desirably, the weight ratio of the aqueous organic solvent and crude rebaudioside A ranges from about 10 to about 4 parts aqueous organic solvent to I part crude rebaudioside A, more particularly from about 5 to about 3 parts aqueous organic solvent to 1 part crude rebaudioside A.

The method of purifying rebaudioside A may be carried out at approximately room temperature. The method of purifying rebaudioside A may further comprises the step of heating the rebaudioside A solution to a temperature in a range from about 20°C to about 40°C, or to a reflux temperature, for about 0.25 hours to about 8 hours. Wherein the method for purifying rebaudioside A comprises the step of heating the rebaudioside A solution, the method may further comprise the step of cooling the rebaudioside A solution to a temperature in the range from about 4°C to about 25°C for about 0.5 hours to about 24 hours.

The purity of rebaudioside A may range from about 50% to about 100%; from about 70% to about 100%; from about 80% to about 100%; from about 90% to about 100%; from about 95% to about 100%; from about 95% to about 99.5%; about 96% to about 100%; from about 97% to about 100%; from about 98% to about 100%; and from about 99% to about 100%. Upon crystallization of crude rebaudioside A, the substantially pure rebaudioside A composition may comprise rebaudioside A in a purity greater than about 95 % by weight up to about 100% by weight on a dry basis. In other exemplary embodiments, substantially pure rebaudioside A comprises purity levels of rebaudioside A greater than about 97 % up to about 100% rebaudioside A by weight on a dry basis, greater than about 98 % up to about 100% by weight on a dry basis, or greater than about 99 % up to about 100% by weight on a dry basis. The rebaudioside A solution during the single crystallization step may be stirred or unstirred.

The method of purifying rebaudioside A may further comprise the step of seeding (optional step) the rebaudioside A solution at an appropriate temperature with high-purity crystals of rebaudioside A sufficient to promote crystallization of the rebaudioside A to form pure rebaudioside A. An amount of rebaudioside A sufficient to promote crystallization of substantially pure rebaudioside A comprises an amount of rebaudioside A from about 0.0001 % to about 1 % by weight of the rebaudioside A present in the solution, more particularly from about 0.01 % to about 1 % by weight. An appropriate temperature for the step of seeding comprises a temperature in a range from about 18°C to about 35°C.

The method of purifying rebaudioside A may further comprise the steps of separating and washing the substantially pure rebaudioside A composition. The substantially pure rebaudioside A composition may be separated from the aqueous organic solution by a variety of solid-liquid separation techniques that utilize centrifugal force, that include, without limitation, vertical and horizontal perforated basket centrifuge, solid bowl centrifuge, decanter centrifuge, peeler type centrifuge, pusher type centrifuge, Heinkel type centrifuge, disc stack centrifuge and cyclone separation. Additionally, separation may be enhanced by any of pressure, vacuum, and gravity filtration methods, that include, without limitation, the use of belt, drum, nutsche type, leaf, plate, Rosenmund type, sparkler type, and bag filters and filter press. Operation of the rebaudioside A solid-liquid separation device may be continuous, semi-continuous or in batch mode. The substantially pure rebaudioside A composition also may be washed on the separation device using various aqueous organic solvents and mixture thereof. The substantially pure rebaudioside A composition can be dried partially or totally on the separation device using any number of gases, including, without limitation, nitrogen and argon, to evaporate residual liquid solvent. The substantially pure rebaudioside A composition may be removed automatically or manually from the separation device using liquids, gases or mechanical means by either dissolving the solid or maintaining the solid form.

The method of purifying rebaudioside A may further comprise the step of drying the substantially pure rebaudioside A composition using techniques well known to those skilled in the art, non-limiting examples of which include the use of a rotary vacuum dryer, fluid bed dryer, rotary tunnel dryer, plate dryer, tray dryer, Nauta type dryer, spray dryer, flash dryer, micron dryer, pan dryer, high and low speed paddle dryer and microwave dryer. The step of drying may comprise drying the substantially pure rebaudioside A composition using a nitrogen or argon purge to remove the residual solvent at a temperature in a range from about 40°C to about 60°C for about 5 hours to about 100 hours.

Wherein the crude rebaudioside A mixture comprises substantially no rebaudioside D impurity, the method of purifying rebaudioside A may further comprise the step of slurrying the composition of substantially pure rebaudioside A with an aqueous organic solvent prior to the step of drying the substantially pure rebaudioside A composition. The slurry is a mixture comprising a solid and an aqueous organic or organic solvent, wherein the solid comprises the substantially pure rebaudioside A composition and is only sparingly soluble in the aqueous organic or organic solvent. The substantially pure rebaudioside A composition and aqueous organic solvent may be present in the slurry in a weight ratio ranging from about 15 parts to 1 part aqueous organic solvent to 1 part substantially pure rebaudioside A composition. The slurry may be maintained at room temperature. The step of slurrying may comprise heating the slurry to a temperature in a range from about 20 to about 40°C. The substantially pure rebaudioside A composition is slurried for about 0.5 hours to about 24 hours.

The method of purifying rebaudioside A may further comprise the steps of separating the substantially pure rebaudioside A composition from the aqueous organic or organic solvent of the slurry and washing the substantially pure rebaudioside A composition followed by the step of drying the substantially pure rebaudioside A composition.

If further purification is desired, the method of purifying rebaudioside A described herein may be repeated or the substantially pure rebaudioside A composition may be purified further using an alternative purification method, such as the column chromatography.

It also is contemplated that other NHPSs may be purified using the purification method described herein, requiring only minor experimentation that would be obvious to those of ordinary skill in the art.

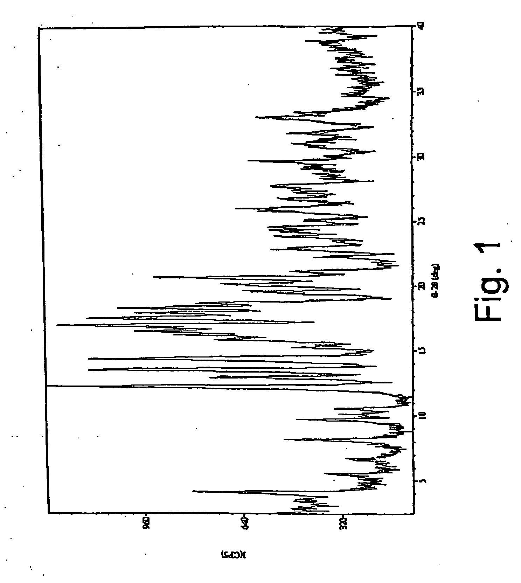

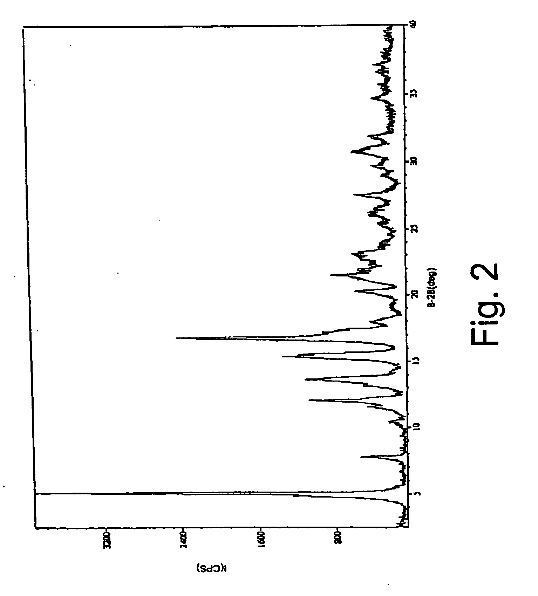

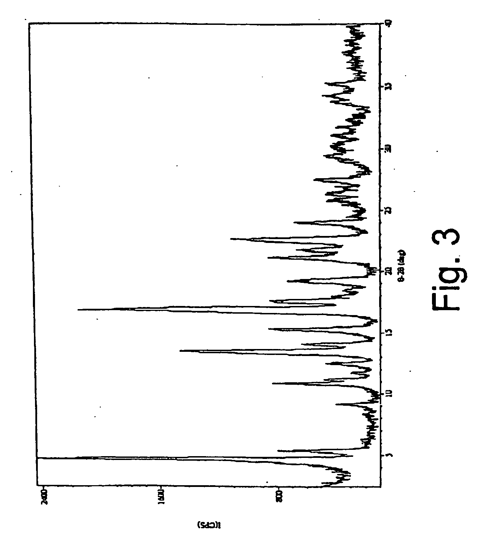

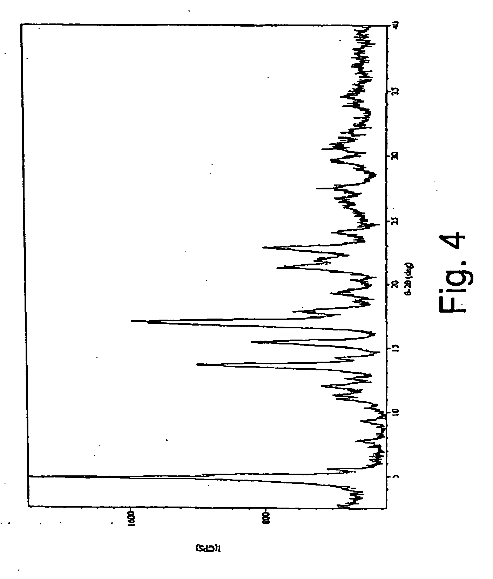

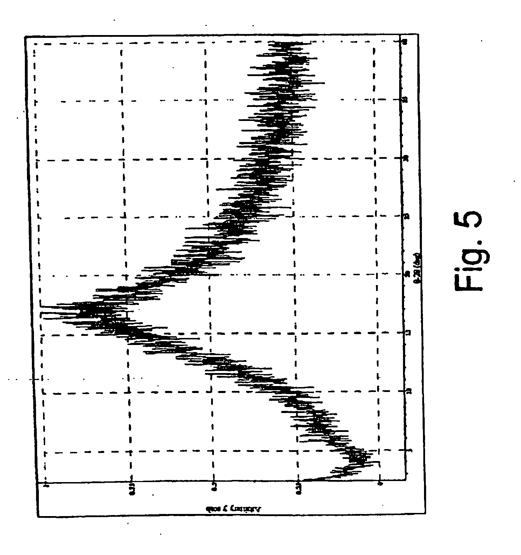

The purification of rebaudioside A by crystallization as described above results in the formation of at least four different polymorphs: Form 1: a rebaudioside A hydrate; Form 2: an anhydrous rebaudioside A; Form 3: a rebaudioside A solvate; and Form 4: an amorphous rebaudioside A. The aqueous organic solution and temperature of the purification process influence the resulting polymorphs in the substantially pure rebaudioside A composition. Figures 1-5 are exemplary powder x-ray diffraction (XRPD) scans of polymorphs Form 1 (hydrate), Form 2 (anhydrate), Form 3A (methanol solvate), Form 3B (ethanol solvate), and Form 4 (amorphous), respectively.

The material properties of the four rebaudioside A polymorphs are summarized in the following table:

**Table 1: Rebaudioside A Polymorphs**

| | **Form 1 Polymorph** | **Form 2 Polymorph** | **Form 3 Polymorph** | **Form 4 Polymorph** |
|---|---|---|---|---|
| Rate of dissolution in H2O at 25°C | Very low (<0.2 %/60 minutes) | Intermediate (<30 %/5 minutes) | High (> 30 % /5 minutes) | High (> 35.0 %/5 minutes) |
| Alcohol content | < 0.5 % | < 1 % | 1-3 % | |
| Moisture content | > 5 % | < 1 % | < 3 % | 6.74 % |

The type of polymorph formed is dependent on the composition of the aqueous organic solution, the temperature of the crystallization step, and the temperature during the drying step. Form 1 and Form 3 are formed during the single crystallization step while Form 2 is formed during the drying step after conversion from Form 1 or Form 3.

Low temperatures during the crystallization step, in the range of about 20°C to about 50°C, and a low ratio of water to the organic solvent in the aqueous organic solvent results in the formation of Form 3. High temperatures during the crystallization step, in the range of about 50°C to about 80°C, and a high ratio of water to the organic solvent in the aqueous organic solvent results in the formation of the Form 1. Form 1 can be converted to Form 3 by slurrying in an anhydrous solvent at room temperature (2-16 hours) or at reflux for approximately (0.5-3 hours). Form 3 can be converted to Form 1 by slurrying the polymorph in water at room temperature for approximately 16 hours or at reflux for approximately 2-3 hours. Form 3 can be converted to the Form 2 during the drying process; however, increasing either the drying temperature above 70°C or the drying time of a substantially pure rebaudioside A composition can result in decomposition of the rebaudioside A and increase the remaining rebaudioside B impurity in the substantially pure rebaudioside A composition. Form 2 can be converted to Form 1 with the addition of water.

Form 4 may be formed from Form 1, 2, 3, or combinations thereof, using methods well known to those of ordinary skill in the art. Non-limiting examples of such methods include melt-processing, ball milling, crystallization, lyophilization, cryo-grinding, and spray-drying. In a particular embodiment, Form 4 can be prepared from a substantially pure rebaudioside A composition obtained by the purification methods described hereinabove by spray-drying a solution of the substantially pure rebaudioside A composition.

As used herein, the phrase "synthetic sweetener" refers to any compositions which are not found in nature and characteristically have a sweetness potency greater than sucrose, fructose, or glucose, yet have less calories. Non-limiting examples of synthetic sweeteners suitable for embodiments of this invention include sucralose, potassium acesulfame, aspartame, alitame, saccharin, neohesperidin dihydrochalcone, cyclamate, neotame, N-[N-[3-(3-hydroxy-4-methoxyphenyl)propyl]-L-α-aspartyl]-L-phenylalanine 1-methyl ester, N-[N-[3-(3-hydroxy-4-methoxyphenyl)-3-methylbutyl]-L-α-aspartyl]-L-phenylalanine 1-methyl ester, N-[N-[3-(3-methoxy-4-hydroxyphenyl)propyl]-L-α-aspartyl)-L-phenylalanine 1-methyl ester, salts thereof, and the like.

The NHPS and synthetic sweeteners may he used individually or in combination with other NHPS and/or synthetic sweeteners. For example, the sweetener composition may comprise a single NHPS or a single synthetic sweetener; a single NHPS in combination with a single synthetic sweetener; one or more NHPSs in combination with a single synthetic sweetener; a single NHPS in combination with one or more synthetic sweeteners; or one or more NHPSs in combination with one or more synthetic sweeteners. A plurality of natural and/or synthetic high-potency sweeteners may be used as long as the combined effect does not adversely affect the taste of the sweetener composition or orally sweetened composition.

### III. Sweet Taste Improving Compositions

The sweetener composition also comprises erythritol.

Combination of rebaudioside A and erythritol suppress, reduce, or eliminate undesirable taste and impart sugar-like characteristics to the sweetener. As used herein, the phrase "undesirable taste" includes any taste property which is not imparted by sugars, e.g. glucose, sucrose, fructose, or similar saccharides. Non-limiting examples of undesirable tastes include delayed sweetness onset, lingering sweet aftertaste, metallic taste, bitter taste, cooling sensation taste or menthol-like taste, licorice-like taste, and/or the like.

A sweetener composition exhibits a more sugar-like temporal and/or sugar-like flavor profile than a sweetener composition comprising rebaudioside A, but without erythritol. As used herein, the phrases "sugar-like characteristic," "sugar-like taste," "sugar-like sweet," "sugary," and "sugar-like" are synonymous. Sugar-like characteristics include any characteristic similar to that of sucrose and include, but are not limited to, maximal response, flavor profile, temporal profile, adaptation behavior, mouthfeel, concentration/response function behavior, tastant and flavor/sweet taste interactions, spatial pattern selectivity, and temperature effects. These characteristics are dimensions in which the taste of sucrose is different from the tastes of natural and synthetic high-potency I sweeteners. Whether or not a characteristic is more sugar-like is determined by expert sensory panel assessments of sugar and compositions comprising at least one natural and/or synthetic high-potency sweetener, both with and without a sweet taste improving composition. Such assessments quantify similarities of the characteristics of compositions comprising at least one natural and/or synthetic high-potency sweetener, both with and without a sweet taste improving composition, with those comprising sugar. Suitable procedures for determining whether a composition has a more sugar-like taste are well known in the art.

In a particular embodiment, a panel of assessors is used to measure the reduction of sweetness linger. Briefly described, a panel of assessors (generally 8 to 12 individuals) is trained to evaluate sweetness perception and measure sweetness at several time points from when the sample is initially taken into the mouth until 3 minutes after it has been expectorated. Using statistical analysis, the results are compared between samples containing additives and samples that do not contain additives. A decrease in score for a time point measured after the sample has cleared the mouth indicates there has been a reduction in sweetness perception.

The panel of assessors may be trained using procedures well known to those of ordinary skill in the art. In a particular embodiment, the panel of assessors may be trained using the Spectrum™ Descriptive Analysis Method (Meilgaard et al, Sensory Evaluation Techniques, 3rd edition, Chapter 11). Desirably, the focus of training should be the recognition of and the measure of the basic tastes; specifcally, sweet. In order to ensure accuracy and reproducibility of results, each assessor should repeat the measure of the reduction of sweetness linger about three to about five times per sample, taking at least a five minute break between each repetition and/or sample and rinsing well with water to clear the mouth.

Generally, the method of measuring sweetness comprises taking a 10mL sample into the mouth, holding the sample in the mouth for 5 seconds and gently swirling the sample in the mouth, rating the sweetness intensity perceived at 5 seconds, expectorating the sample (without swallowing following expectorating the sample), rinsing with one mouthful of water (e.g., vigorously moving water in mouth as if with mouth wash) and expectorating the rinse water, rating the sweetness intensity perceived immediately upon expectorating the rinse water, waiting 45 seconds and, while wating those 45 seconds, identifying the time of maximum perceived sweetness intensity and rating the sweetness intensity at that time (moving the mouth normally and swallowing as needed), rating the sweetness intensity after another 10 seconds, rating the sweetness intensity after another 60 seconds (cumulative 120 seconds after rinse), and rating the sweetness intensity after still another 60 seconds (cumulative 180 seconds after rinse). Between samples take a 5 minute break, rinsing well with water to clear the mouth.

As used herein, the term "carbohydrate" generally refers to aldehyde or ketone compounds substituted with multiple hydroxyl groups, of the general formula (CH₂O)ₙ, wherein n is 3-30, as well as their oligomers and polymers. The carbohydrates of the present invention can, in addition, be substituted or deoxygenated at one or more positions. Carbohydrates, as used herein, encompass unmodified carbohydrates, carbohydrate derivatives, substituted carbohydrates, and modified carbohydrates. As used herein, the phrases "carbohydrate derivatives", "substituted carbohydrate", and "modified carbohydrates" are synonymous. Modified carbohydrate means any carbohydrate wherein at least one atom has been added, removed, substituted, or combinations thereof. Thus, carbohydrate derivatives or substituted carbohydrates include substituted and unsubstituted monosaccharides, disaccharides, oligosaccharides, and polysaccharides. The carbohydrate derivatives or substituted carbohydrates optionally can be deoxygenated at any corresponding C-position, and/or substituted with one or more moieties such as hydrogen, halogen, haloalkyl, carboxyl, acyl, acyloxy, amino, amido, carboxyl derivatives, alkylamino, dialkylamino, arylamino, alkoxy, aryloxy, nitro, cyano, sulfo, mercapto, imino, sulfonyl, sulfenyl, sulfinyl, sulfamoyl, carboalkoxy, carboxamido, phosphonyl, phosphinyl, phosphoryl, phosphino, thioester, thioether, oximino, hydrazino, carbamyl, phospho, phosphonato, or any other viable functional group provided the carbohydrate derivative or substituted carbohydrate functions to improve the sweet taste of at least one natural and/or synthetic high-potency sweetener.

Non-limiting examples of carbohydrates include tagatose, trehalose, galactose, rhamnose, cyclodextrin (e.g., α-cyclodextrin, β-cyclodextrin, and γ-cyclodextrin), maltodextrin (including resistant maltodextrins such as Fibersol-2™), dextran, sucrose, glucose, ribulose, fructose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, amylopectin, glucosamine, mannosamine, fucose, glucuronic acid, gluconic acid, glucono-lactone, abequose, galactosamine, beet oligosaccharides, isomalto-oligosaccharides (isomaltose, isomaltotriose, panose and the like), xylo-oligosaccharides (xylotriose, xylobiose and the like), gentio-oligoscaccharides (gentiobiose, gentiotriose, gentiotetraose and the like), sorbose, nigero-oligosaccharides, palatinose oligosaccharides, fructooligosaccharides (kestose, nystose and the like), maltotetraol, maltotriol, malto-oligosaccharides (maltotriose, maltotetraose, maltopentaose, maltohexaose, maltoheptaose and the like), lactulose, melibiose, raffinose, rhamnose, ribose, isomerized liquid sugars such as high fructose corn/starch syrup (e.g., HFCS55, HFCS42, or HFCS90), coupling sugars, soybean oligosaccharides, and glucose syrup. Additionally, the carbohydrates as used herein may be in either the D- or L- configuration.

The term "polyol", as used herein, refers to a molecule that contains more than one hydroxyl group. A polyol may be a diol, triol, or a tetraol which contain 2, 3, and 4 hydroxyl groups, respectively. A polyol also may contain more than four liydroxyl groups, such as a pentaol, hexaol, heptaol, or the like, which contain, 5, 6, or 7 hydroxyl groups, respectively. Additionally, a polyol also may be a sugar alcohol, polyhydric alcohol, or polyalcohol which is a reduced form of carbohydrate, wherein the carbonyl group (aldehyde or ketone, reducing sugar) has been reduced to a primary or secondary hydroxyl group.

Non-limiting examples of sweet taste improving polyol additives include maltitol, mannitol, sorbitol, lactitol, xylitol, inositol, isomalt, propylene glycol, glycerol (glycerine), threitol, galactitol, palatinose, reduced isomalto-oligosaccharides, reduced xylo-oligosaccharides, reduced gentio-oligosaccharides, reduced maltose syrup, reduced glucose syrup, and sugar alcohols or any other carbohydrates capable of being reduced which do not adversely affect the taste of the at least one natural and/or synthetic high-potency sweetener or the orally ingestible composition.

Suitable sweet taste improving amino acid additives for use include, but are not limited to, aspartic acid, arginine, glycine, glutamic acid, proline, threonine, theanine, cysteine, cystine, alanine, valine, tyrosine, leucine, isoleucine, asparagine, serine, lysine, histidine, ornithine, methionine, carnitine, aminobutyric acid (alpha-, beta-, or gamma-isomers), glutamine, hydroxyproline, taurine, norvaline, sarcosine, and their salt forms such as sodium or potassium salts or acid salts. The sweet taste improving amino acid additives also may be in the D- or L- configuration and in the mono-, di-, or tri- form of the same or different amino acids. Additionally, the amino acids may be α-, β-, γ-, δ-, and ε- isomers if appropriate. Combinations of the foregoing amino acids and their corresponding salts (e.g., sodium, potassium, calcium, magnesium salts or other alkali or alkaline earth metal salts thereof, or acid salts) also are suitable sweet taste improving additives. The amino acids may be natural or synthetic. The amino acids also may be modified. Modified amino acids refers to any amino acid wherein at least one atom has been added, removed, substituted, or combinations thereof (e.g., N-alkyl amino acid, N-acyl amino acid, or N-methyl amino acid). Non-limiting examples of modified amino acids include amino acid derivatives such as trimethyl glycine, N-methyl-glycine, and N-methyl-alanine. As used herein, amino acids encompass both modified and unmodified amino acids. As used herein, modified amino acid also may encompass peptides and polypeptides (e.g., dipeptides, tripeptides, tetrapeptides, and pentapeptides) such as glutathione and L-alanyl-L-glutamine.

Suitable sweet taste improving polyamino acid additives include poly-L-aspartic acid, poly-L-lysine (e.g., poly-L-α-lysine or poly-L-ε-lysine), poly-L-ornithine (e.g., poly-L-α-ornithine or poly-L-ε-ornithine), poly-L-arginine, other polymeric forms of amino acids, and salt forms thereof (e.g., magnesium, calcium, potassium, or sodium salts such as L-glutamic acid mono sodium salt). The sweet taste improving polyamino acid additives also may be in the D- or L- configuration. Additionally, the polyamino acids may be α-, β-, γ-, δ-, and ε- isomers if appropriate. Combinations of the foregoing polyamino acids and their corresponding salts (e.g., sodium, potassium, calcium, magnesium salts or other alkali or alkaline earth metal salts thereof or acid salts) also are suitable sweet taste improving additives. The polyamino acids described herein also may comprise copolymers of different amino acids. The polyamino acids may be natural or synthetic. The polyamino acids also may be modified, such that at least one atom has been added, removed, substituted, or combinations thereof (e.g., N-alkyl polyamino acid or N-acyl polyamino acid). As used herein, polyamino acids encompass both modified and unmodified polyamino acids. In accordance with particular embodiments, modified polyamino acids include, but are not limited to polyamino acids of various molecular weights (MW), such as poly-L-α-lysine with a MW of 1,500, MW of 6,000, MW of ) 25,200, MW of 63,000, MW of 83,000, or MW of 300,000.

Suitable sweet taste improving sugar acid additives for use include, but are not limited to, aldonic, uronic, aldaric, alginic, gluconic, glucuronic, glucaric, galactaric, galacturonic, and their salts (e.g., sodium, potassium, calcium, magnesium salts or other physiologically acceptable salts), and combinations thereof.

Suitable sweet taste improving nucleotide additives for use include, but are not limited to, inosine monophosphate ("IMP"), guanosine monophosphate ("GMP"), adenosine monophosphate ("AMP"), cytosine monophosphate (CMP), uracil monophosphate (UMP), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, and their alkali or alkaline earth metal salts, and combinations thereof. The nucleotides described herein also may comprise nucleotide-related additives, such as nucleosides or nucleic acid bases (e.g., guanine, cytosine, adenine, thymine, uracil).

Suitable sweet taste improving organic acid additives include any compound which comprises a -COOH moiety. Suitable sweet taste improving organic acid additives for use include, but are not limited to, C2-C30 carboxylic acids, substituted hydroxyl C1-C30 carboxylic acids, benzoic acid, substituted benzoic acids (e.g. 2,4-dihydroxybenzoic acid), substituted cinnamic acids, hydroxyacids, substituted hydroxybenzoic acids, substituted cyclohexyl carboxylic acids, tannic acid, lactic acid, tartaric acid, citric acid, gluconic acid, glucoheptonic acids, adipic acid, hydroxycitric acid, malic acid, fruitaric acid (a blend of malic, fumaric, and tartaric acids), fumaric acid, maleic acid, succinic acid, chlorogenic acid, salicylic acid, creatine, glucosamine hydrochloride, glucono delta lactone, caffeic acid, bile acids, acetic acid, ascorbic acid, alginic acid, erythorbic acid, polyglutamic acid, and their alkali or alkaline earth metal salt derivatives thereof. In addition, the sweet taste improving organic acid additives also may be in either the D- or L- configuration.

Suitable sweet taste improving organic acid salt additives include, but are not limited to, sodium, calcium, potassium, and magnesium salts of all organic acids, such as salts of citric acid, malic acid, tartaric acid, fumaric acid, lactic acid (e.g., sodium lactate), alginic acid (e.g., sodium alginate), ascorbic acid (e.g., sodium ascorbate), benzoic acid (e.g., sodium benzoate or potassium benzoate), and adipic acid. The examples of the sweet taste improving organic acid salt additives described optionally may be substituted with one or more of the following moiety selected from the group consisting of hydrogen, alkyl, alkenyl, alkynyl, halo, haloalkyl, carboxyl, acyl, acyloxy, amino, amido, carboxyl derivatives, alkylamino, dialkylamino, arylamino, alkoxy, aryloxy, nitro, cyano, sulfo, thiol, imine, sulfonyl, sulfenyl, sulfinyl, sulfamyl, carboxalkoxy, carboxamido, phosphonyl, phosphinyl, phosphoryl, phosphino, thioester, thioether, anhydride, oximino, hydrazino, carbamyl, phospho, phosphonato, and any other viable functional group, provided the substituted organic acid salt additive functions to improve the sweet taste of the at least one natural and/or synthetic high-potency sweetener.

Suitable sweet taste improving inorganic acid additives for use include, but are not limited to, phosphoric acid, phosphorous acid, polyphosphoric acid, hydrochloric acid, sulfuric acid, carbonic acid, sodium dihydrogen phosphate, and their corresponding alkali or alkaline earth metal salts thereof (e.g., inositol hexaphosphate Mg/Ca).

Suitable sweet taste improving bitter compound additives for use include, but are not limited to, caffeine, quinine, urea, bitter orange oil, naringin, quassia, and salts thereof.

Suitable sweet taste improving flavorant and flavoring ingredient additives for use include, but are not limited to, vanillin, vanilla extract, mango extract, cinnamon, citrus, coconut, ginger, viridiflorol, almond, menthol (including menthol without mint), grape skin extract, and grape seed extract. "Flavorant'' and "flavoring ingredient" are synonymous, and include natural or synthetic substances or combinations thereof. Flavorants also include any other substance which imparts flavor, and may include natural or non-natural (synthetic) substances which are safe for human or animals when used in a generally accepted range. Non-limiting examples of proprietary flavorants include Döhler™ Natural Flavoring Sweetness Enhancer K14323 (Döhler™, Darmstadt, Germany), Symrise™ Natural Flavor Mask for Sweeteners 161453 and 164126 (Symrise, Holzminden™, Germany), Natural Advantage™ Bitterness Blockers 1, 2, 9 and 10 (Natural Advantage™, Freehold, New Jersey, U.S.A.), and Sucramask™ (Creative Research Management, Stockton, California, U.S.A.).

Suitable sweet taste improving polymer additives for use include, but are not limited to, chitosan, pectin, pectic, pectinic, polyuronic, polygalacturonic acid, starch, food hydrocolloid or crude extracts thereof (e.g., gum acacia senegal (Fibergum™), gum acacia seyal, carageenan), poly-L-lysine (e.g., poly-L-α-lysine or poly-L-ε-lysine), poly-L-ornithine (e.g., poly-L-α-ornithine or poly-L-ε-ornithine), polyarginine, polypropylene glycol, polyethylene glycol, poly(ethylene glycol methyl ether), polyaspartic acid, polyglutamic acid, polyethyleneimine, alginic acid, sodium alginate, propylene glycol alginate, sodium hexametaphosphate (SHMP) and its salts, and sodium polyethyleneglycolalginate and other cationic and anionic polymers.

Suitable sweet taste improving protein or protein hydrolysate additives for use in embodiments of this invention include, but are not limited to, bovine serum albumin (BSA), whey protein (including fractions or concentrates thereof such as 90% instant whey protein isolate, 34% whey protein, 50% hydrolyzed whey protein, and 80% whey protein concentrate), soluble rice protein, soy protein, protein isolates, protein hydrolysates, reaction products of protein hydrolysates, glycoproteins, and/or proteoglycans containing amino acids (e.g., glycine, alanine, serine, threonine, asparagine, glutamine, arginine, valine, isoleucine, leucine, norvaline, methionine, proline, tyrosine, hydroxyproline, and the like), collagen (e.g., gelatin), partially hydrolyzed collagen (e.g., hydrolyzed fish collagen), and collagen hydrolysates (e.g., porcine collagen hydrolysate).

Suitable sweet taste improving surfactant additives for use include, but are not limited to, polysorbates (e.g., polyoxyethylene sorbitan monooleate (polysorbate 80), polysorbate 20, polysorbate 60), sodium dodecylbenzenesulfonate, dioctyl sulfosuccinate or dioctyl sulfosuccinate sodium, sodium dodecyl sulfate, cetylpyridinium chloride (hexadecylpyridinium chloride), hexadecyltrimethylammonium bromide, sodium cholate, carbamoyl, choline chloride, sodium glycocholate, sodium taurodeoxycholate, lauric arginate, sodium stearoyl lactylate, sodium taurocholate, lecithins, sucrose oleate esters, sucrose stearate esters, sucrose palmitate esters, sucrose laurate esters, and other emulsifiers, and the like.

Suitable sweet taste improving flavonoid additives for use generally are classified as flavonols, flavones, flavanones, flavan-3-ols, isoflavones, or anthocyanidins. Non-limiting examples of flavonoid additives include catechins (e.g., green tea extracts such as Polyphenon™ 60, Polyphenon™ 30, and Polyphenon™ 25 (Mitsui Norin Co., Ltd., Japan), polyphenols, rutins (e.g., enzyme modified rutin Sanmelin™ AO (San-Ei Gen F.F.I., Inc., Osaka, Japan)), neohesperidin, naringin, neohesperidin dihydrochalcone, and the like.

Suitable sweet taste improving alcohol additives for use include, but are not limited to, ethanol.

Suitable sweet taste improving astringent compound additives include, but are not limited to, tannic acid, europium chloride (EuCl₃), gadolinium chloride (GdCl₃), terbium chloride (TbCl₃), alum; tannic acid, and polyphenols (e.g., tea polyphenols).

Suitable sweet taste improving vitamins include nicotinamide (Vitamin B3) and pyridoxal hydrochloride (Vitamin B6).

The sweet taste improving compositions also may comprise other natural and/or synthetic high-potency sweeteners. For example, wherein the functional sweetener composition comprises at least one NHPS, the at least one sweet taste improving composition may comprise a synthetic high-potency sweetener, non-limiting examples of which include sucralose, potassium acesulfame, aspartame, alitame, saccharin, neohesperidin dihydrochalcone, cyclamate, neotame, N-[N-[3-(3-hydroxy-4-methoxyphenyl)propyl]-L-α-aspartyl]-L-phenylalanine 1-methyl ester, N-[N-[3-(3-hydroxy-4-methoxyphenyl)-3-methylbulyl]-L-α-aspartyl]-L-phenylalanine 1-methyl ester, N-[N-[3-(3-methoxy-4-hydroxyphenyl)propyl]-L-α-aspartyl]-L-phenylalanine 1-methyl ester, salts thereof, and the like.

The sweet taste improving compositions also may be in salt form which may be obtained using standard procedures well known in the art. The term "salt" also refers to complexes that retain the desired chemical activity of the sweet taste improving compositions of the present invention and are safe for human or animal consumption in a generally acceptable range. Alkali metal (for example, sodium or potassium) or alkaline earth metal (for example, calcium or magnesium) salts also can be made. Salts also may include combinations of alkali and alkaline earth metals. Non-limiting examples of such salts are (a) acid addition salts formed with inorganic acids and salts formed with organic acids; (b) base addition salts formed with metal cations such as calcium, bismuth, barium, magnesium, aluminum, copper, cobalt, nickel, cadmium, sodium, potassium, and the like, or with a cation formed from ammonia, N,N-dibenzylethylenediamine, D-glucosamine, tetraethylammonium, or ethylenediamine; or (c) combinations of (a) and (b). Thus, any salt forms which may be derived from the sweet taste improving compositions may be used as long as the salts of the sweet taste improving additives do not adversely affect the taste of the at least one natural and/or synthetic high-potency sweeteners or the orally ingestible compositions comprising the at least one natural and/or synthetic high-potency sweetener. The salt forms of the additives can be added to the natural and/or synthetic sweetener composition in the same amounts as their acid or base forms.

Suitable sweet taste improving inorganic salts useful as sweet taste improving additives include, but are not limited to, sodium chloride, potassium chloride, sodium sulfate, potassium citrate, europium chloride (EuCl₃), gadolinium chloride (GdCl₃), terbium chloride (TbCl₃), magnesium sulfate, alum, magnesium chloride, mono-, di-, tribasic sodium or potassium salts of phosphoric acid (e.g., inorganic phosphates), salts of hydrochloric acid (e.g., inorganic chlorides), sodium carbonate, sodium bisulfate, and sodium bicarbonate. Furthermore, suitable organic salts useful as sweet taste improving additives include, but are not limited to, choline chloride, alginic acid sodium salt (sodium alginate), glucoheptonic acid sodium salt, gluconic acid sodium salt (sodium gluconate), gluconic acid potassium salt (potassium gluconate), guanidine HCI, glucosamine HCI, amiloride HCI, monosodium glutamate (MSG), adenosine monophosphate salt, magnesium gluconate, potassium tartrate (monohydrate), and sodium tartrate (dihydrate).

It has been discovered that combinations of rebaudioside A and erythritol improve the temporal profile and/or flavor profile, including the osmotic taste, to be more sugar-like.

According to a particular embodiment of this invention, the functional sweetener composition provided herein comprises erythritol in an amount effective for the functional sweetener composition to impart an osmolarity of at least 10 mOsmoles/L to an aqueous solution of the functional sweetener composition, wherein the rebaudioside A is present in the aqueous solution in an amount sufficient to impart a maximum sweetness intensity equivalent to that of a 10% aqueous solution of sucrose by weight. As used herein, "mOsmoles/L" refers to milliosmoles per liter. According to another embodiment, the functional sweetener composition comprises erythritol in an amount effective for the functional sweetener composition to impart an osmolarity of 10 to 500 mOsmoles/L, preferably 25 to 500 mOsmoles/L preferably, more preferably 100 to 500 mOsmoles/L, more preferably 200 to 500 mOsmoles/L, and still more preferably 300 to 500 mOsmoles/L to an aqueous solution of the functional sweetener composition, wherein the rebaudioside A is present in the aqueous solution in an amount sufficient to impart a maximum sweetness intensity equivalent to that of a 10% aqueous solution of sucrose by weight. Wherein a plurality of sweet taste improving compositions are combined with at least one natural and/or synthetic high-potency sweetener and at least one functional ingredient, the osmolarity imparted is that of the total combination of the plurality of sweet taste improving compositions.

Osmolarity refers to the measure of osmoles of solute per liter of solution, wherein osmole is equal to the number of moles of osmotically active particles in an ideal solution (e.g., a mole of glucose is one osmole), whereas a mole of sodium chloride is two osmoles (one mole of sodium and one mole of chloride). Thus, in order to improve in the quality of taste of the functional sweetener composition, the osmotically active compounds or the compounds which impart osmolarity must not introduce significant off taste to the formulation.

Suitable sweet taste improving carbohydrate additives have a molecular weight less than or equal to 500 and desirably have a molecular weight from 50 to 500. Suitable carbohydrates with a molecular weight less than or equal to 500 include, but are not limited to, sucrose, fructose, glucose, maltose, lactose, mannose, galactose, and tagatose. Crenerally, a sweet taste improving carbohydrate additive may be present in the functional sweetener compositions in an amount from about 1,000 to about 100,000 ppm. (Throughout this specification, the term ppm means parts per million by weight or volume. For example, 500 ppm means 500 mg in a liter.) A sweet taste improving carbohydrate additive may be present in the sweetened compositions in an amount from about 2,500 to about 10,000 ppm. Suitable sweet taste improving carbohydrate additives for imparting osmolarities ranging from about 10 mOsmoles/L to about 500 mOsmoles/L to a sweetenable composition include, but are not limited to, sweet taste improving carbohydrate additives with a molecular weight ranging from about 50 to about 500.

The erythritol is present in sweetener compositions in an amount from about about 5,000 to about 40,000 ppm, and still more particularly from about 10,000 to about 35,000 ppm.

A suitable sweet taste improving alcohol additive is present in the functional sweetener compositions in an amount from about 625 to about 10,000 ppm. Suitable sweet taste improving alcohol additives for imparting osmolarities ranging from about 10 mOsmoles/L to about 500 mOsmoles/L to a sweetenable composition include, but are not limited to, sweet taste improving alcohol additives with a molecular weight ranging from about 46 to about 500. A non-limiting example of sweet taste improving alcohol additive with a molecular weight ranging from about 46 to about 500 includes ethanol.

Suitable sweet taste improving amino acid additives have a molecular weight of less than or equal to 250 and desirably have a molecular weight from 75 to 250. Suitable sweet taste improving amino acid additives with a molecular weight less than or equal to 250 include, but are not limited to, glycine, alanine, serine, valine, leucine, isoleucine, proline, theanine, and threonine. Preferred sweet taste improving amino acid additives include those which are sweet tasting at high concentrations, but desirably are present in embodiments of this invention at amounts below or above their sweetness taste detection threshold. Even more preferred are mixtures of sweet taste improving amino acid additives at amounts below or above their sweetness taste detection threshold. Generally, a sweet taste improving amino acid additive may be present in the functional sweetener compositions in an amount from about 100 ppm to about 25,000 ppm, more particularly from about 1,000 to about 10,000 ppm, and still more particularly from about 2,500 to about 5,000 ppm. A sweet taste improving amino acid additive may be present in the sweetened compositions in an amount from about 250 ppm to about 7,500 ppm. Suitable sweet taste improving amino acid additives for imparting osmolarities ranging from about 10 mOsmoles/L to about 500 mOsmoles/L to a sweetenable composition include, but are not limited to, sweet taste improving amino acid additives with a molecular weight ranging from about 75 to about 250.

Generally, a suitable sweet taste improving amino acid salt additive may be present in the functional sweetener compositions in an amount from about 25 to about 10,000 ppm, more particularly from about 1,000 to about 7,500 ppm, and still more particularly from about 2,500 to about 5,000 ppm. Suitable sweet taste improving amino acid salt additives for imparting osmolarities ranging from about 10 mOsmoles/L to about 500 mOsmoles/L to a sweetenable composition include, but are not limited to, sweet taste improving amino acid salt additives with a molecular weight ranging from about 75 to about 300. Non-limiting examples of sweet taste improving amino acid salt additives with a molecular weight ranging from about 75 to about 300 include salts of glycine, alanine, serine, theanine, and threonine.

Generally, a suitable sweet taste improving protein or protein hydroyslate additive may be present in the functional sweetener compositions in an amount from about 200 to about 50,000 ppm. Suitable sweet taste improving protein or protein hydrolysate additives for imparting osmolarities ranging from about 10 mOsmoles/L to about 500 mOsmoles/L to a sweetenable composition include, but are not limited to, sweet taste improving protein or protein hydrolysate additives with a molecular weight ranging from about 75 to about 300. Non-limiting examples of sweet taste improving protein or protein hydrolysate additives with a molecular weight ranging from about 75 to about 300 include proteins or protein hydrolysates containing glycine, alanine, serine, and threonine.

Generally, a suitable sweet taste improving inorganic acid additive may be present in the functional sweetener compositions in an amount from about 25 to about 5,000 ppm. Suitable sweet taste improving inorganic acid additives for imparting osmolarities ranging from about 10 mOsmoles/L to about 500 mOsmoles/L to a sweetenable composition include, but are not limited to, phosphoric acid, HCl, and H₂SO₄ and any other inorganic acid additives which are safe for human or animal consumption when used in a generally acceptable range. Suitable sweet taste improving inorganic acid additives for imparting osmolarities ranging from about 10 mOsmoles/L to about 500 mOsmoles/L to a sweetenable composition include, but are not limited to, sweet taste improving inorganic acid additives with a molecular weight range from about 36 to about 98.

Generally, a suitable sweet taste improving inorganic acid salt additive may be present in the functional sweetener compositions in an amount from about 25 to about 5,000 ppm. Suitable sweet taste improving inorganic acid salt additives for imparting osmolarities ranging from about 10 mOsmoles/L to about 500 mOsmoles/L to a sweetenable composition include, but are not limited to, salts of inorganic acids, for example sodium, potassium, calcium, and magnesium salts of phosphoric acid, and any other alkali or alkaline earth metal salts of other inorganic acids (e.g., sodium bisulfate) which are safe for human or animal consumption when used in a generally acceptable range. Suitable suitable sweet taste improving inorganic acid salt additives for imparting osmolarities ranging from about 10 mOsmoles/L to about 500 mOsmoles/L to a sweetenable composition include, but are not limited to, sweet taste improving inorganic acid salt additives with a molecular weight range from about 58 to about 120.

Generally, a suitable sweet taste improving organic acid additive may be present in the functional sweetener compositions in an amount from about 10 to about 5,000 ppm. Suitable sweet taste improving organic acid additives for imparting osmolarities ranging from about 10 mOsmoles/L to about 500 mOsmoles/L to a sweetenable composition include, but are not limited to, creatine, citric acid, malic acid, succinic acid, hydroxycitric acid, tartaric acid, fumaric acid, gluconic acid, glutaric acid, adipic acid, and any other sweet taste improving organic acid additives which are safe for human or animal consumption when used in a generally acceptable range. The sweet taste improving organic acid additive may comprise a molecular weight range from about 60 to about 208.

Generally, a suitable sweet taste improving organic acid salt additive may be present in the functional sweetener compositions in an amount from about 20 to about 10,000 ppm. Suitable sweet taste improving organic acid salt additives for imparting osmolarities ranging from about 10 mOsmoles/L to about 500 mOsmoles/L to a sweetenable composition include, but are not limited to, salts of sweet taste improving organic acid additives, such as sodium, potassium, calcium, magnesium, and other alkali or alkaline metal salts of citric acid, malic acid, tartaric acid, fumaric acid, gluconic acid, glutaric acid, adipic acid, hydroxycitric acid, succinic acid, and salts of any other sweet taste improving organic acid additives which are safe for human or animal consumption when used in a generally acceptable range. The sweet taste improving organic acid salt additive may comprises a molecular weight range from about 140 to about 208.

Generally, a suitable sweet taste improving organic base salt additive may be present in the functional sweetener compositions in an amount from about 10 to about 5,000 ppm. Suitable sweet taste improving organic base salt additives for imparting osmolarities ranging from about 10 mOsmoles/L to about 500 mOsmoles/L to a sweetenable composition include, but are not limited to, inorganic and organic acid salts of organic bases such as glucosamine salts, choline salts, and guanidine salts.

Generally, a suitable sweet taste improving astringent additive may be present in the functional sweetener compositions in an amount from about 25 to about 1,000 ppm. Suitable sweet taste improving astringent additives for imparting osmolarities ranging from about 10 mOsmoles/L to about 500 mOsmoles/L to a sweetenable composition include, but are not limited to, tannic acid, tea polyphenols, catechins, aluminum sulfate, AlNₐ(SO₄)₂, AlK(SO₄)₂ and other forms of alum.

Generally, a suitable sweet taste improving nucleotide additive may be present in the functional sweetener compositions in an amount from about 5 to about 1,000 ppm. Suitable sweet taste improving nucleotide additives for imparting osmolarities ranging from about 10 mOsmoles/L to about 500 mOsmoles/L to a sweetenable composition include, but are not limited to, adenosine monophosphate.

Generally, a suitable sweet taste improving polyamino acid additive may be present in the functional sweetener compositions in an amount from about 30 to about 2,000 ppm. Suitable sweet taste improving polyamino acid additives for imparting osmolarities ranging from about 10 mOsmoles/L to about 500 mOsmoles/L to a sweetenable composition include, but are not limited to, poly-L-lysine (e.g., poly-L-α-lysine or poly-1,-ε-lysine), poly-L-ornithine (e.g., poly-L-α-ornithine or poly-L-ε-ornithine), and poly-L-arginine.

Generally, a suitable sweet taste improving polymer additive may be present in the functional sweetener compositions in an amount from about 30 to about 2,000 ppm. Suitable sweet taste improving polymer additives for imparting osmolarities ranging from about 10 mOsmoles/L to about 500 mOsmoles/L to a sweetenable composition include, but are not limited to, chitosan, sodium hexametaphosphate and its salts, pectin, hydrocolloids such as gum acacia senegal, propylene glycol, polyethylene glycol, and poly(ethylene glycol methyl ether).

Generally, a suitable sweet taste improving surfactant additive may be present in the functional sweetener compositions in an amount from about 1 to about 5,000 ppm. Suitable sweet taste improving surfactant additives for imparting osmolarities ranging from about 10 mOsmoles/L to about 500 mOsmoles/L to a sweetenable composition include, but are not limited to, polysorbates, choline chloride, sodium taurocholate, lecithins, sucrose oleate esters, sucrose stearate esters, sucrose palmitate esters, and sucrlose laurate esters.

Generally, a suitable sweet taste improving flavonoid additive may be present in the functional sweetener compositions in an amount from about 0.1 to about 1,000 ppm. Suitable sweet taste improving flavonoid additives for imparting osmolarities ranging from about 10 mOsmoles/L to about 500 mOsmoles/L to a sweetenable composition include, but are not limited to, naringin, catechins, rutins, neohesperidin, and neohesperidin dihydrochalcone.

Suitable sweet taste improving carbohydrate additives for improving the osmotic taste of the natural and/or synthetic high-potency sweetener to be more sugar-like include, but are not limited to, sweet taste improving carbohydrate additives with a molecular weight ranging from about 50 to about 500. Non-limiting examples of sweet taste improving carbohydrate additives with a molecular weight ranging from about 50 to about 500 include sucrose, fructose, glucose, maltose, lactose, mannose, galactose, ribose, rhamnose, trehalose, HFCS, and tagatose.

Suitable sweet taste improving polyol additives for improving the osmotic taste of natural and/or synthetic high-potency sweetener to be more sugar-like include, but are not limited to, sweet taste improving polyol additives with a molecular weight ranging from about 76 to about 500. Non-limiting examples of sweet taste improving polyol additives with a molecular weight ranging from about 76 to about 500 include erythritol, glycerol, and propylene glycol. Other suitable sweet taste improving polyol additives may include sugar alcohols.

Suitable sweet taste improving alcohol additives for improving the osmotic taste of natural and/or synthetic high-potency sweetener to be more sugar-like include, but are not limited to, sweet taste improving alcohol additives with a molecular weight ranging from about 46 to about 500. A non-limiting example of sweet taste improving alcohol additive with a molecular weight ranging from about 46 to about 500 includes ethanol.

Suitable sweet taste improving amino acid additives for improving the osmotic taste of natural and/or synthetic high-potency sweetener to be more sugar-like include, but are not limited to, sweet taste improving amino acid additives with a molecular weight ranging from about 75 to about 250. Non-limiting examples of sweet taste improving amino acid additives with a molecular weight ranging from about 75 to about 250 include glycine, alanine, serine, leucine, valise, isoleucine, proline, hydroxyproline, glutamine, theanine, and threonine.

Suitable sweet taste improving amino acid salt additives for improving the osmotic taste of natural and/or synthetic high-potency sweetener to be more sugar-like include, but are not limited to, sweet taste improving amino acid salt additives with a molecular weight ranging from about 75 to about 300. Non-limiting examples of sweet taste improving amino acid salt additives with a molecular weight ranging from about 75 to about 300 include salts of glycine, alanine, serine, leucine, valine, isoleucine, proline, hydroxyproline, glutamine, theanine, and threonine.

Suitable sweet taste improving protein or protein hydrolysate additives for improving the osmotic taste of natural and/or synthetic high-potency sweetener to be more sugar-like include, but are not limited to, sweet taste improving protein or protein hydrolysate additives with a molecular weight ranging from about 75 to about 300. Non-limiting examples of sweet taste improving protein or protein hydrolysate additives with a molecular weight ranging from about 75 to about 300 include protein or protein hydrolysates containing glycine, alanine, serine, leucine, valine, isoleucine, proline, and threonine.

Suitable sweet taste improving inorganic acid salt additives for improving the osmotic taste of natural and/or synthetic high-potency sweetener to be more sugar-like include, but are not limited to, sodium chloride, potassium chloride, magnesium chloride, KH₂PO₄ and NaH₂PO₄. Suitable sweet taste improving inorganic acid salt additives for improving the osmotic taste may comprise a molecular weight from about 58 to about 120.

Suitable sweet taste improving bitter additives for improving the osmotic taste of the natural and/or synthetic high-potency sweetener to be more sugar-like include, but are not limited to, caffeine, quinine, urea, quassia, tannic acid, and naringin.

A functional sweetener composition is provided comprising at least one functional ingredient and at least one natural and/or synthetic high-potency sweetener in combination with at least one sweet taste improving nucleotide additive chosen from inosine monophosphate ("IMP"), guanosine monophosphate ("GMP"), adenosine monophosphate ("AMP"), cytosine monophosphate (CMP), uracil monophosphate (UMP), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, nucleosides thereof, nucleic acid bases thereof, or salts thereof.

A functional sweetener composition is provided comprising at least one functional ingredient and at least one natural and/or synthetic high-potency sweetener in combination with at least one sweet taste improving carbohydrate additive chosen from tagatose, trehalose, galactose, rhamnose, cyclodextrin (e.g., α-cyclodextrin, β-cyclodextrin, and γ-cyclodextrin), maltodextrin (including resistant maltodextrins such as Fibersol-2™), dextran, sucrose, glucose, ribulose, fructose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, amylopectin, glucosamine, mannosamine, fucose, glucuronic acid, gluconic acid, glucono-lactone, abequose, galactosamine, beet oligosaccharides, isomalto-oligosaccharides (isomaltose, isomaltotriose, panose and the like), xylo-oligosaccharides (xylotriose, xylobiose and the like), gentio-oligoscaccharides (gentiobiose, geatiotriose, gentiotetraose and the like), sorbose, nigero-oligosaccharides, palatinose oligosaccharides, fucose, fructooligosaccharides (kestose, nystose and the like), maltotetraol, maltotriol, malto-oligosaccharides (maltotriose, maltotetraose, maltopentaose, maltohexaose, maltoheptaose and the like), lactulose, melibiose, raffinose, rhamnose, ribose, isomerized liquid sugars such as high fructose corn/starch syrup (e.g., HFCS55, HFCS42, or HFCS90), coupling sugars, soybean oligosaccharides, or glucose syrup.

In another embodiment, a functional sweetener composition is provided comprising rebaudioside A and.

A functional sweetener composition is provided comprising at least one functional ingredient and at least one natural and/or synthetic high-potency sweetener in combination with at least one sweet taste improving amino acid additive chosen from aspartic acid, arginine, glycine, glutamic acid, proline, threonine, theanine, cysteine, cystine, alanine, valine, tyrosine, leucine, isoleucine, asparagine, serine, lysine, histidine, ornithine, methionine, carnitine, aminobutyric acid (alpha-, beta-, and gamma-isomers), glutamine, hydroxyproline, taurine, norvaline, sarcosine, or salts thereof.

A functional sweetener composition is provided comprising at least one functional ingredient and at least one natural and/or synthetic high-potency sweetener in combination with at least one sweet taste improving polyamino acid additive chosen from poly-L-aspartic acid, poly-L-lysine (e.g., poly-L-α-lysine or poly-L-ε-lysine), poly-L-ornithine (e.g., poly-L-α-ornithine or poly-L-ε-ornithine), poly-L-arginine, other polymeric forms of amino acids, or salts thereof.

A functional sweetener composition is provided comprising at least one functional ingredient and at least one natural and/or synthetic high-potency sweetener in combination with at least one sweet taste improving sugar acid additive chosen from aldonic, uronic, aldaric, alginic, gluconic, glucuronic, glucaric, galactaric, galacturonic, or salts thereof.

A functional sweetener composition is provided comprising at least one functional ingredient and at least one natural and/or synthetic high-potency sweetener in combination with at least one sweet taste improving organic acid additive chosen from C2-C30 carboxylic acids, substituted hydroxyl C1-C30 carboxylic acids, benzoic acid, substituted benzoic acids (e.g., 2,4-dihydroxybenzoic acid), substituted cinnamic acids, hydroxyacids, substituted hydroxybenzoic acids, substituted cyclohexyl carboxylic acids, tannic acid, lactic acid, tartaric acid, citric acid, gluconic acid, glucoheptonic acids, glutaric acid, creatine, adipic acid, hydroxycitric acid, malic acid, fruitaric acid, fumaric acid, maleic acid, succinic acid, chlorogenic acid, salicylic acid, caffeic acid, bile acids, acetic acid, ascorbic acid, alginic acid, erythorbic acid, polyglutamic acid, or salts thereof.

A functional sweetener composition is provided comprising at least one functional ingredient and at least one natural and/or synthetic high-potency sweetener in combination with at least one sweet taste improving inorganic acid additive chosen from phosphoric acid, phosphorous acid, polyphosphoric acid, hydrochloric acid, sulfuric acid, carbonic acid, sodium dihydrogen phosphate, or salts thereof.

A functional sweetener composition is provided comprising at least one functional ingredient and at least one natural and/or synthetic high-potency sweetener in combination with at least one sweet taste improving bitter compound additive chosen from caffeine, quinine, urea, bitter orange oil, naringin, quassia, or salts thereof.

A functional sweetener composition is provided comprising at least one functional ingredient and at least one natural and/or synthetic high-potency sweetener in combination with at least one sweet taste improving flavorant additive chosen from vanillin, vanilla extract, mango extract, cinnamon, citrus, coconut, ginger, viridiflorol, almond, menthol, grape skin extract, or grape seed extract. In another particular embodiment, the at least one sweet taste improving flavorant additive comprises a proprietary sweetener chosen from Döhler™ Natural Flavoring Sweetness Enhancer K14323 (Döhler™, Darmstadt, Germany), Symrise™ Natural Flavor Mask for Sweeteners 161453 or 164126 (Symrise™, Holzminden, Germany), Natural Advantage™ Bitterness Blockers 1, 2, 9 or 10 (Natural Advantage™, Freehold, New Jersey, U.S.A.), or Sucramask™ (Creative Research Management, Stockton, California, U.S.A.)

A functional sweetener composition is provided comprising at least one functional ingredient and at least one natural and/or synthetic high-potency sweetener in combination with at least one sweet taste improving polymer additive chosen from chitosan, pectin, pectic, pectinic, polyuronic, polygalacturonic acid, starch, food hydrocolloid or crude extracts thereof (e.g., gum acacia senegal, gum acacia seyal, carageenan), poly-L-lysine (e.g., poly-L-α-lysine or poly-L-ε-lysine), poly-L-omithine (e.g., poly-L-α-ornithine or poly-L-ε-ornithine), polypropylene glycol, polyethylene glycol, poly(ethylene glycol methyl ether), polyarginine, polyaspartic acid, polyglutamic acid, polyethyleneimine, alginic acid, sodium alginate, propylene glycol alginate, sodium polyethyleneglycolalginate, sodium hexametaphosphate and its salts, or other cationic and anionic polymers.

A functional sweetener composition is provided comprising at least one functional ingredient and at least one natural and/or synthetic high-potency sweetener in combination with at least one sweet taste improving protein hydrolysate additive chosen from bovine serum albumin (BSA), whey protein (including fractions or concentrates thereof such as 90% instant whey protein isolate, 34% whey protein, 50% hydrolyzed whey protein, and 80% whey protein concentrate), soluble rice protein, soy protein, protein isolates, protein hydrolysates, reaction products of protein hydrolysates, glycoproteins, and/or proteoglycans containing amino acids (e.g., glycine, alanine, serine, threonine, theanine, asparagine, glutamine, arginine, valine, isoleucine, leucine, norvaline, methionine, proline, tyrosine, hydroxyproline, or the like).

A functional sweetener composition is provided comprising at least one functional ingredient and at least one natural and/or synthetic high-potency sweetener in combination with at least one sweet taste improving surfactant additive chosen from polysorbates (e.g., polyoxyethylene sorbitan monooleate (polysorbate 80), polysorbate 20, polysorbate 60), sodium dodecylbenzenesulfonate, dioctyl sulfosuccinate or dioctyl sulfosuccinate sodium, sodium dodecyl sulfate, cetylpyridinium chloride, hexadecyltrimethylammonium bromide, sodium cholate, carbamoyl, choline chloride, sodium glycocholate, sodium taurocholate, sodium taurodeoxycholate, lauric arginate, sodium stearoyl lactylate, lecithins, sucrose oleate esters, sucrose stearate esters, sucrose palmitate esters, sucrose laurate esters, and other emulsifiers, or the like.

A functional sweetener composition is provided comprising at least one functional ingredient and at least one natural and/or synthetic high-potency sweetener in combination with at least one sweet taste improving flavonoid additive chosen from catechins, polyphenols, rutins, neohesperidin, naringin, neohesperidin dihydrochalcone, or the like.

A functional sweetener composition is provided comprising at least one functional ingredient and at least one natural and/or synthetic high-potency sweetener in combination with ethanol.

A functional sweetener composition is provided comprising at least one functional ingredient and at least one natural and/or synthetic high-potency sweetener in combination with at least one sweet taste improving astringent compound additive chosen from tannic acid, europium chloride (EuCl₃), gadolinium chloride (GdCl₃), terbium chloride (TbCl₃), alum, tannic acid, and polyphenols (e.g., tea polyphenol).

A functional sweetener composition is provided comprising at least one functional ingredient and at least one natural and/or synthetic high-potency sweetener in combination with at least one sweet taste improving inorganic salt additive chosen from sodium chloride, potassium chloride, sodium dihydrogen phosphate, sodium sulfate, potassium citrate, europium chloride (EuCl₃), gadolinium chloride (GdCl₃), terbium chloride (TbCl₃), magnesium sulfate, magnesium phosphate, alum, magnesium chloride, mono-, di-, tri-basic sodium or potassium salts of phosphoric acid, salts of hydrochloric acid, sodium carbonate, sodium bisulfate, or sodium bicarbonate.

A functional sweetener composition is provided comprising at least one functional ingredient and at least one natural and/or synthetic high-potency sweetener in combination with at least one sweet taste improving organic salt additive chosen from choline chloride, gluconic acid sodium salt, gluconic acid potassium salt, guanidine HCl, amiloride HCl, glucosamine HCl, monosodium glutamate (MSG), adenosine monophosphate salt, magnesium gluconate, potassium tartrate, and sodium tartrate.

A functional sweetener composition is provided comprising at least one functional ingredient and at least one natural and/or synthetic high-potency sweetener in combination with at least one sweet taste improving nucleotide additive, at least one sweet taste improving carbohydrate additive, and at least one sweet taste improving amino acid additive; wherein the at least one nucleotide additive is chosen from inosine monophosphate ("IMP"), guanosine monophosphate ("GMP''), adenosine monophosphate ("AMP"), cytosine monophosphate (CMP), uracil monophosphate (UMP), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, nucleosides thereof, nucleic acid bases thereof, or salts thereof; wherein the at least one carbohydrate additive is chosen from tagatose, trehalose, galactose, rhamnose, cyclodextrin (e.g., α-cyclodextrin, β-cyclodextrin, and γ-cyclodextrin), maltodextrin (including resistant maltodextrins such as Fibersol-2™), dextran, sucrose, glucose, ribulose, fructose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, amylopectin, glucosamine, mannosamine, fucose, glucuronic acid, gluconic acid, glucono-lactone, abequose, galactosamine, beet oligosaccharides, isomalto-oligosacharides (isomaltose, isomaltotriose, panose and the like), xylo-oligosaccharides (xylotriose, xylobiose and the like), gentio-oligoscaccharides (gentiobiose, gentiotriose, gentiotetraose and the like), sorbose, nigero-oligosaccharides, palatinose oligosaccharides, fucose, fructooligosaccharides (kestose, nystose and the like), maltotetraol, maltotriol, malto-oligosaccharides (maltotriose, maltotetraose, maltopentaose, maltohexaose, maltoheptaose and the like), lactulose, melibiose, raffinose, rhamnose, ribose, isomerized liquid sugars such as high fructose corn/starch syrup (e.g., HFCS55, HFCS42, or HFCS90), coupling sugars, soybean oligosaccharides, or glucose syrup; and wherein the at least one amino acid additive is chosen from aspartic acid, arginine, glycine, glutamic acid, proline, threonine, theanine, cysteine, cystine, alanine, valine, tyrosine, leucine, isoleucine, asparagine, serine, lysine, histidine, ornithine, methionine, carnitine, aminobutyric acid (alpha-, beta-, and gamma-isomers), glutamine, hydroxyproline, taurine, norvaline, sarcosine, or salts thereof.

A functional sweetener composition is provided comprising at least one functional ingredient and at least one natural and/or synthetic high-potency sweetener in combination with at least one sweet taste improving nucleotide additive and at least one sweet taste improving carbohydrate additive; wherein the at least one nucleotide additive is chosen from inosine monophosphate (''IMP"), guanosine monophosphate ("GMP"), adenosine monophosphate ("AMP''), cytosine monophosphate (CMP), uracil monophosphate (UMP), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, nucleosides thereof, nucleic acid bases thereof, or salts thereof; and wherein the at least one carbohydrate additive is chosen from tagatose, trehalose, galactose, rhamnose, cyclodextrin (e.g., α-cyclodextrin, β-cyclodextrin, and γ-cyclodextrin), maltodextrin (including resistant maltodextrins such as Fibersol-2™), dextran, sucrose, glucose, ribulose, fructose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, amylopectin, glucosamine, mannosamine, fucose, glucuronic acid, gluconic acid, glucono-lactone, abequose, galactosamine, beet oligosaccharides, isomalto-oligosaccharides (isomaltose, isomaltotriose, panose and the like), xylo-oligosaccharides (xylotriose, xylobiose and the like), gentio-oligoscaccharides (gentiobiose, gentiotriose, genetiotetraose and the like), sorbose, nigero-oligosaccharides, palatinose oligosaccharides, fucose, fructooligasaccharides (kestose, nystose and the like), maltotetraol, maltotriol, malto-oligosaccharides (maltotriose, maltotetraose, maltopentaose, maltohexaose, maltohepcaose and the like), lactulose, melibiose, raffinose, rhamnose, ribose, isomerized liquid sugars such as high fructose corn/starch syrup (e.g., HFCS55, HFCS42, or HFCS90), coupling sugars, soybean oligosaccharides, or glucose syrup.

A functional sweetener composition is provided comprising at least one functional ingredient and rebaudioside A with at least one sweet taste improving nucleotide additive and erythritol; wherein the at least one nucleotide additive is chosen from inosine monophosphate ("IMP"), guanosine monophosphate ("GMP"), adenosine monophosphate ("AMP"), cytosine monophosphate (CMP), uracil monophosphate (UMP), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, nucleosides thereof, nucleic acid bases thereof, or salts thereof.

A functional sweetener composition is provided comprising at least one functional ingredient and at least one natural and/or synthetic high-potency sweetener in combination with at least one sweet taste improving nucleotide additive and at least one sweet taste improving amino acid; wherein the at least one nucleotide additive is chosen from inosine monophosphate ("IMP"), guanosine monophosphate ("GMP"), adenosine monophosphate ("AMP"), cytosine monophosphate (CMP), uracil monophosphate (UMP), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, nucleosides thereof, nucleic acid bases thereof, or salts thereof; and wherein the at least one amino acid additive is chosen from aspartic acid, arginine, glycine, glutamic acid, proline, threonine, theanine, cysteine, cystine, alanine, valine, tyrosine, leucine, isoleucine, asparagine, serine, lysine, histidine, ornithine, methionine, carnitine, aminobutyric acid (alpha-, beta-, and gamma-isomers), glutamine, hydroxyproline, taurine, norvaline, sarcosine, or salts thereof.

A functional sweetener composition is provided comprising at least one functional ingredient and rebaudioside A with at least one sweet taste improving carbohydrate additive, erythritol, and at least one sweet taste improving amino acid additive; wherein the at least one carbohydrate additive is chosen from tagatose, trehalose, galactose, rhamnose, cyclodextrin (e.g., α-cyclodextrin, β-cyclodextrin, and γ-cyclodextrin), maltodextrin (including resistant maltodextrins such as Fibersol-2™), dextran, sucrose, glucose, ribulose, fructose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, amylopectin, glucosamine, mannosamine, fucose, glucuronic acid, gluconic acid, glucono-lactone, abequose, galactosamine, beet oligosaccharides, isomalto-oligosaccharides (isomaltose, isomaltotriose, panose and the like), xylo-oligosaccharides (xylotriose, xylobiose and the like), gentio-oligoscaccharides (gentiobiose, gentiotriose, gentiotetraose and the like), sorbose, nigero-oligosaccharides, palatinose oligosaccharides, fucose, fructooligosaccharides (kestose, nystose and the like), maltotetraol, maltotriol, malto-oligosaccharides (maltotriose, maltotetraose, maltopentaose, maltohexaose, maltoheptaose and the like), lactulose, melibiose, raffinose, rhamnose, ribose, isomerized liquid sugars such as high fructose corn/starch syrup (e.g., HFCS55, HFCS42, or HFCS90), coupling sugars, soybean oligosaccharides, or glucose syrup; and wherein the at least one amino acid additive is chosen from aspartic acid, arginine, glycine, glutamic acid, proline, threonine, theanine, cysteine, cystine, alanine, valine, tyrosine, leucine, isoleucine, asparagine, serine, lysine, histidine, ornithine, methionine, carnitine, aminobutyric acid (alpha-, beta-, and gamma-isomers), glutamine, hydroxyproline. taurine, norvaline, sarcosine, or salts thereof.

A functional sweetener composition is provided comprising at least one functional ingredient and rebaudioside A in combination with at least one sweet taste improving carbohydrate additive and erythritol; wherein the at least one carbohydrate additive is chosen from tagatose, trehalose, galactose, rhamnose, cyclodextrin (e.g., α-cyclodextrin, β-cyclodextrin, and γ-cyclodextrin), maltodextrin (including resistant maltodextrins such as Fibersol-2™), dextran, sucrose, glucose, ribulose, fructose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, amylopectin, glucosamine, mannosamine, fucose, glucuronic acid, gluconic acid, glucono-lactone, abequose, galactosamine, beet oligosaccharides, isomalto-oligosaccharides (isomaltose, isomaltotriose, panose and the like), xylo-oligosaccharides (xylotriose, xylobiose and the like), gentio-oligoscaccharides (gentiobiose, gentiotriose, gentiotetraose and the like), sorbose, nigero-oligosaccharides, palatinose oligosaccharides, fucose, fructooligosaccharides (kestose, nystose and the like), maltotetraol, maltotriol, malto-oligosaccharides (maltotriose, maltotetraose, maltopentaose, maltohexaose, maltoheptaose and the like), lactulose, melibiose, raffinose, rhamnose, ribose, isomerized liquid sugars such as high fructose corn/starch syrup (e.g., HFCS55, HFCS42, or HFCS90), coupling sugars, soybean oligosaccharides, or glucose syrup.

A functional sweetener composition is provided comprising at least one functional ingredient and at least one natural and/or synthetic high-potency sweetener in combination with at least one sweet taste improving carbohydrate additive and at least one sweet taste improving amino acid additive; wherein the at least one carbohydrate additive is chosen from tagatose, trehalose, galactose, rhamnose, cyclodextrin (e.g., α-cyclodextrin, β-cyclodextrin, and γ-cyclodextrin), maltodextrin (including resistant maltodextrins such as Fibersol-2™), dextran, sucrose, glucose, ribulose, fructose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, amylopectin, glucosamine, mannosamine, fucose, glucuronic acid, gluconic acid, glucono-lactone, abequose, galactosamine, beet oligosaccharides, isomalto-oligosaccharides (isomaltose, isomaltotriose, panose and the like), xylo-oligosaccharides (xylotriose, xylobiose and the like), gentio-oligoscaccharides (gentiobiose, gentiotriose, gentiotetraose and the like), sorbose, nigero-oligosaccharides, palatinose oligosaccharides, fucose, fructooligosaccharides (kestose, nystose and the like), maltotetraol, maltotriol, malto-oligosaccharides (maltotriose, maltotetraose, maltopentaose, maltohexaose, maltoheptaose and the like), lactulose, melibiose, raffinose, rhamnose, ribose, isomerized liquid sugars such as high fructose corn/starch syrup (e.g., HFCS55, HFCS42, or HFCS90), coupling sugars, soybean oligosaccharides, or glucose syrup; and wherein the at least one amino acid additive is chosen from aspartic acid, arginine, glycine, glutamic acid, proline, threonine, theanine, cysteine, cystine, alanine, valine, tyrosine, leucine, isoleucine, asparagine, serine, lysine, histidine, ornithine, methionine, carnitine, aminobutyric acid (alpha-, beta-, and gamma-isomers), glutamine, hydroxyproline, taurine, norvaline, sarcosine, or salts thereof.

A functional sweetener composition is provided comprising at least one functional ingredient and rebaudioside A in combination with erythritol and at least one sweet taste improving amino acid additive; and wherein the at least one amino acid additive is chosen from aspartic acid, arginine, glycine, glutamic acid, proline, threonine, theanine, cysteine, cystine, alanine, valine, tyrosine, leucine, isoleucine, asparagine, serine, lysine, histidine, ornithine, methionine, carnitine, aminobutyric acid (alpha-, beta-, and gamma-isomers), glutamine, hydroxyproline, taurine, norvaline, sarcosine, or salts thereof.

Aa functional sweetener composition is provided comprising at least one functional ingredient and rebaudioside A in combination with erythritol and at least one sweet taste improving inorganic salt additive; and wherein the at least one inorganic salt additive is chosen from sodium chloride, potassium chloride, sodium dihydrogen phosphate, sodium sulfate, potassium citrate, europium chloride (EuCl₃), gadolinium chloride (GdCl₃), terbium chloride (TbCl₃), magnesium sulfate, alum, magnesium chloride, mono-, di-, tri-basic sodium or potassium salts of phosphoric acid, salts of hydrochloric acid, sodium carbonate, sodium bisulfate, or sodium bicarbonate.

A functional sweetener composition is provided comprising at least one functional ingredient and at least one natural and/or synthetic high-potency sweetener in combination with at least one sweet taste improving carbohydrate additive and at least one sweet taste improving inorganic salt additive; wherein the at least one carbohydrate additive is chosen from tagatose, trehalose, galactose, rhamnose, cyclodextrin (e.g., α-cyclodextrin, β-cyclodextrin, and γ-cyclodextrin), maltodextrin (including resistant maltodextrins such as Fibersol-2™); dextran, sucrose, glucose, ribulose, fructose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, amylopectin, glucosamine, mannosamine, fucose, glucuronic acid, gluconic acid, glucono-lactone, abequose, galactosamine, beet oligosaccharides, isomalto-oligosaccharides (isomaltose, isomaltotriose, panose and the like), xylo-oligosaccharides (xylotriose, xylobiose and the like), gentio-oligoscaccharides (gentiobiose, gentiotriose, gentiotetraose and the like), sorbose, nigero-oligosaccharides, palatinose oligosaccharides, fucose, fructooligosaccharides (kestose, nystose and the like), maltotetraol, maltotriol, malto-oligosaccharides (maltotriose, maltotetraose, maltopentaose, maltohexaose, maltoheptaose and the like), lactulose, melibiose, raffinose, rhamnose, ribose, isomerized liquid sugars such as high fructose corn/starch syrup (e.g., HFCS55, HFCS42, or HFCS90), coupling sugars, soybean oligosaccharides, or glucose syrup; and wherein the at least one inorganic salt additive is chosen from sodium chloride, potassium chloride, sodium dihydrogen phosphate, sodium sulfate, potassium citrate, europium chloride (EuCl₃), gadolinium chloride (GdCl₃), terbium chloride (TbCl₃), magnesium phosphate, magnesium sulfate, alum, magnesium chloride, mono-, di-, tri-basic sodium or potassium salts of phosphoric acid, salts of hydrochloric acid, sodium carbonate, sodium bisulfate, or sodium bicarbonate.

A functional sweetener composition is provided comprising at least one functional ingredient and at least one natural and/or synthetic high-potency sweetener in combination with at least one sweet taste improving carbohydrate additive, at least one sweet taste improving amino acid additive, and at least one sweet taste improving inorganic salt additive; wherein the at least one carbohydrate additive is chosen from tagatose, trehalose, galactose, rhamnose, cyclodextrin (e.g., α-cyclodextrin, β-cyclodextrin, and γ-cyclodextrin), maltodextrin (including resistant maltodextrins such as Fibersol-2™), dextran, sucrose, glucose, ribulose, fructose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, eryrthrulose, xylulose, psicose, turanose, cellobiose, amylopectin, glucosamine, mannosamine, fucose, glucuronic acid, gluconic acid, glucono-lactone, abequose, galactosamine, beet oligosaccharides, isomalto-oligosaccharides (isomaltose, isomaltotriose, panose and the like), xylo-oligosaccharides (xylotriose, xylobiose and the like), gentio-oligoscaccharides (gentiobiose, gentiotriose, gentiotetraose and the like), sorbose, nigero-oligosaccharides, palatinose oligosaccharides, fucose, fructooligosaccharides (kestose, nystose and the like), maltotetraol, maltotriol, malto-oligosaccharides (maltotriose, maltotetraose, maltopentaose, maltohexaose, maltoheptaose and the like), lactulose, melibiose, raffinose, rhamnose, ribose, isomerized liquid sugars such as high fructose corn/starch syrup (e.g., HFCS55, HFCS42, or HFCS90), coupling sugars, soybean oligosaccharides, or glucose syrup; wherein the at least one amino acid additive is chosen from aspartic acid, arginine, glycine, glutamic acid, proline, threonine, theanine, cysteine, cystine, alanine, valine, tyrosine, leucine, isoleucine, asparagine, serine, lysine, histidine, ornithine, methionine, carnitine, aminobutyric acid (alpha-, beta-, and gamma-isomers), glutamine, hydroxyproline, taurine, norvaline, sarcosine, or salts thereof; and wherein the at least one inorganic salt additive is chosen from sodium chloride, potassium chloride, sodium sulfate, potassium citrate, europium chloride (EuCl₃), gadolinium chloride (GdCl₃), terbium chloride (TbCl₃), magnesium phosphate, magnesium sulfate, alum, magnesium chloride, mono-, di-, tri-basic sodium or potassium salts of phosphoric acid, salts of hydrochloric acid, sodium carbonate, sodium bisulfate, or sodium bicarbonate.

In another embodiment, a functional sweetener composition is provided comprising at least one functional ingredient and rebaudioside A in combination with erythritol and at least one sweet taste improving polyamino acid additive; and wherein the at least one polyamino acid additive is chosen from poly-L-aspartic acid, poly-L-lysine (e.g., poly-L-α-lysine or poly-L-ε-lysine), poly-L-ornithine (e.g., poly-L-α-ornithine or poly-L-ε-ornithine), poly-L-arginine, and other polymeric forms of amino acids, or salts thereof.

A functional sweetener composition is provided comprising at least one functional ingredient and at least one natural and/or synthetic high-potency sweetener in combination with at least one sweet taste improving protein or protein hydrolysate additive and at least one sweet taste improving inorganic salt additive; wherein the at least one sweet taste improving protein or protein hydrolysate additive is chosen from bovine serum albumin (BSA), whey protein (including fractions or concentrates thereof such as 90% instant whey protein isolate, 34% whey protein, 50% hydrolyzed whey protein, and 80% whey protein concentrate), soluble rice protein, soy protein, protein isolates, protein hydrolysates, reaction products of protein hydrolysates, glycoproteins, and/or proteoglycans containing amino acids (e.g., glycine, alanine, serine, threonine, theanine, asparagine, glutamine, arginine, valine, isoleucine, leucine, norvaline, methionine, proline, tyrosine, hydroxyproline, or the like), collagen (e.g., gelatin), partially hydrolyzed collagen (e.g., hydrolyzed fish collagen), and collagen hydrolysates (e.g., porcine collagen hydrolysate); and wherein the at least one sweet taste improving inorganic salt additive is chosen from sodium chloride, potassium chloride, sodium sulfate, potassium citrate, europium chloride (EuCl₃), gadolinium chloride (GdCl₃), terbium chloride (TbCl₃), magnesium phosphate, magnesium sulfate, alum, magnesium chloride, mono-, di-, tribasic sodium or potassium salts of phosphoric acid, salts of hydrochloric acid, sodium carbonate, sodium bisulfate, or sodium bicarbonate.

A functional sweetener composition is provided comprising at least one functional ingredient and rebaudioside A in combination with at least one natural and/or synthetic high-potency sweetener other than rebaudioside-A and at least one sweet taste improving composition.

A functional sweetener composition is provided comprising at least one functional ingredient and rebaudioside A in combination with at least one synthetic high-potency sweetener, wherein the at least one synthetic high-potency sweetener functions as a sweet taste improving composition. Non-limiting examples of suitable sweet taste improving synthetic sweetener additives include sucralose, potassium acesulfame, aspartame, alitame, saccharin, neohesperidin dihydrochalcone, cyclamate, neotame, N-[N-[3-(3-hydroxy-4-methoxyphenyl)propyl]-L-α-aspartyl]-L-phenylalanine 1-methyl ester, N-[N-[3-(3-hydroxy-4-methoxyphenyl)-3-methylbutyl]-L-α-aspartyl]-L-phenylalanine 1-methyl ester, N-[N-[3-(3-methoxy-4-hydroxyphenyl)propyl]-L-α-aspartyl]-L-phenylalanine 1-methyl ester, salts thereof, and the like.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, cyclamate, saccharin, aspartame, acesulfame potassium or other salts, or neotame, in combination with at least one sweet taste improving amino acid additive and erythritol is provided. The at least one sweet taste improving amino acid additive may be present in an amount from about 100 ppm to about 25,000 ppm of the composition, and the erythritol is present in an amount from about 400 to about 80,000 ppm of the composition. The at least one sweet taste improving amino acid additive may be glycine or alanine.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving amino acid additive and at least one sweet taste improving protein or protein hydrolysate additive is provided. The at least one sweet taste improving amino acid additive may be present in an amount from about 100 to about 25,000 ppm of the composition, and the at least one sweet taste improving protein or protein hydrolysate additive may be present in an amount from about 200 ppm to about 50,000 ppm of the composition. The at least one sweet taste improving amino acid additive may be glycine or lysine, and the at least one sweet taste improving protein or protein hydrolysate additive may be a protein, a hydrolysate, or a reaction product of a hydrolysate of a protein containing glycine, alanine, serine, leucine, valine, isoleucine, proline, or threonine.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving protein or protein hydrolysate additive and at least one sweet taste improving polyol additive is provided. The at least one sweet taste improving protein or protein hydrolysate additive may be present in an amount from about 200 ppm to about 50,000 ppm of the composition, and at least one sweet taste improving polyol additive may be present in an amount from about 400 to about 80,000 ppm of the composition. The at least one sweet taste improving protein or protein hydrolysate additive may be a protein, a hydrolysate, or a reaction product of a hydrolysate of proteins containing glycine, alanine, serine, leucine, valine, isoleucine, proline, or threonine.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving carbohydrate additive is provided. The at least one sweet taste improving carbohydrate additive may be present in an amount from about 1,000 to about 100,000 ppm of the composition. The composition comprises REBA and glucose, sucrose, HFCS, or D-fructose may be in an amount from about 10,000 ppm to about 80,000 ppm of the composition.

In one embodiment, a functional sweetener composition comprising rebaudioside-A (REBA) (with at least 50 % REBA in a steviol glycoside mixture) in combination with erythritol is provided. In a particular embodiment of the functional sweetener composition, rebaudioside A is present in an amount from about 100 to about 3,000 ppm and the erythritol is present in an amount from about 400 to about 80,000 ppm of the total sweetener composition. In another embodiment of the functional sweetener composition, rebaudioside A is present in an amount from about 100 to about 3,000 ppm and the erythritol is present in an amount from about 5,000 to about 40,000 ppm of the total sweetener composition. In still another embodiment of the functional sweetener composition, rebaudioside A is present in an amount from about 100 to about 3,000 ppm and the erythritol is present in an amount from about 10,000 to about 35,000 ppm of the total sweetener composition. In another particular embodiment of the functional sweetener composition, rebaudioside A and erythritol are present in the sweetener composition in a ratio from about 1:4 to about 1:800, respectively. In yet another particular embodiment of the functional sweetener composition, rebaudioside A and erythritol are present in the sweetener composition in a ratio from about 1:20 to about 1:600, respectively; more particularly from about 1:50 to about 1:300; and still more particularly from about 1:75 to about 1:150.

A functional sweetener composition comprising at least one functional ingredient and a sweetener composition comprising rebaudioside-A (REBA), stevia, stevioside, mogroside IV, mogroside V, Luo Han Guo sweetener, monatin, or curculin, in combination with at least one sweet taste improving synthetic sweetener additive is provided. The functional sweetener composition may comprises at least one functional ingredient and a sweetener comprising rebaudioside-A (REBA) in combination with saccharin or acesulfame potassium or other salts in an amount from about 10 ppm to about 100 ppm of the composition.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving carbohydrate additive and at least one sweet taste improving polyol additive is provided. The at least one sweet taste improving carbohydrate additive may be present in an amount from about 1,000 to about 100,000 ppm of the composition and at least one sweet taste improving polyol additive is present in an amount from about 400 to about 80,000 ppm of the composition. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with tagatose, fructose or sucrose and erythritol.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving inorganic salt additive is provided. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with NaCl, KCl, NaHSO₄.H2O, NaH₂PO₄, MgSO₄, KAl(SO₄)₂ (alum), magnesium phosphate, magnesium chloride, KCl and KH₂PO₄, or other combinations thereof. A particularly desirable embodiment comprises the at least one functional ingredient and a sweetener comprising rebaudioside-A (REBA), stevia, stevioside, mogroside IV, mogroside V, Luo Han Guo sweetener, (monatin, curculin, sucralose, saccharin, cyclamate, aspartame, acesulfame potassium or other salts, or neotame, in combination with a mixture of inorganic salt additives, such as chlorides, phosphates, and sulfates of sodium, magnesium, potassium, and calcium (e.g., sodium chloride and potassium chloride; potassium phosphate and potassium chloride; sodium chloride and sodium phosphate; calcimn phosphate and calcium sulfate; magnesium chloride and magnesium phosphate; and calcium phosphate, calcium sulfate, and potassium sulfate).

A functional sweetener composition comprising at least one functional ingredient and a sweetener comprises aspartame, acesfulame potassium or other salts, and sucralose in combination with at least one sweet taste improving inorganic salt additive. The at least one sweet taste improving inorganic salt additive may be is present in an amount in the range of about 25 to about 5,000 ppm of the composition. Non-limiting examples include at least one functional ingredient and a sweetener comprising aspartame, acesulfame potassium, and sucralose in combination with magnesium chloride; at least one functional ingredient and a sweetener comprising aspartame, acesulfame potassium, and sucralose in combination with magnesium sulfate; or at least one functional ingredient and a sweetener comprising aspartame, acesulfame potassium, and sucralose in combination with magnesium sulfate and sodium chloride.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving organic acid salt additive is provided. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with choline chloride in citrate buffer, D-gluconic acid sodium salt, guanidine HCl, D-glucosamine HCl, amiloride HCl, or combinations thereof.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving organic acid additive is provided. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with fumaric acid, malic acid, tartaric acid, citric acid, adipic acid, ascorbic acid, tannic acid, succinic acid, glutaric acid, or combinations thereof.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving amino acid additive is provided. The at least one sweet taste improving amino acid additive may be present in an amount from about 100 to about 25,000 ppm of the composition. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with glycine, L-alanine, L-serine, L-threoone, β-alanine, aminobutyric acid (alpha-, beta-, or gamma-isomers), L-aspartic acid, L-glutamic acid, L-lysine, glycine and L-alanine mixture, salt derivatives or combinations thereof.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving surfactant additive is provided. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with dioctyl sulfosuccinate sodium, cetylpyridinium chloride, hexadecyltrimethylammonium bromide, sucrose oleate, polysorbate 20, polysorbate 80, lecithin, or combinations thereof.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving polymer additive is provided. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with cationic polymer such as polyethyleneimine, poly-L-lysine (e.g., poly-L-α-lysine or poly-L-ε-lysine), poly-L-ornithine (e.g., poly-L-α-ornithine or poly-L-ε-ornithine), chitosan, or combinations thereof.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving polymer additive and at least one sweet taste improving polyol additive is provided. The at least one sweet taste improving polymer additive may be present in an amount from about 30 to about 2,000 ppm of the composition, and the at least one sweet taste improving polyol additive is present in an amount from about 400 to about 80,000 ppm of the composition. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with a hydrocolloid, such as a gum acacia seyal, and erythritol.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving protein or protein hydrolysate additive is provided. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with bovine serum albumin (BSA), whey protein or combinations thereof.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving amino acid additive and at least one sweet taste improving inorganic acid salt additive is provided. The at least one sweet taste improving amino acid additive may be present in an amount from about 100 to about 25,000 ppm of the composition and the at least one sweet taste improving inorganic acid salt additive is present in an amount from about 25 to about 5,000 ppm of the composition. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with glycine and alum; rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with glycine and potassium chloride; rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with glycine and sodium chloride; REBA in combination with glycine, potassium dihydrogen phosphate, and potassium chloride; and rebaudioside-A (REBA), stevia, stevioside, morgroside IV, morgroside V, Lo Han Guo, monatin, curculin, sucralose, saccharin, aspartame, acesulfame potassium or other salts, or neotame, in combination with glycine, sodium chloride, and potassium chloride.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving carbohydrate additive and at least one sweet taste improving inorganic acid salt additive is provided. The at least one sweet taste improving carbohydrate additive may be present in an amount from about 1,000 to about 100,000 ppm of the composition and the at least one sweet taste improving inorganic acid salt additive may be present in an amount from about 25 ppm to about 5,000 ppm. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with fructose, sucrose, or glucose and alum; at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with fructose, sucrose, or glucose and potassium chloride; at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with fructose, sucrose, or glucose and sodium chloride; at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with fructose, sucrose, or glucose, potassium phosphate, and potassium chloride; and at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with fructose, sucrose, or glucose, sodium chloride, and potassium chloride.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving bitter additive and at least one sweet taste improving inorganic salt additive is provided. A non-limiting example include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with urea and sodium chloride.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving amino acid additive and at least one sweet taste improving polyamino acid additive is provided. The at least one sweet taste improving amino acid additive may be present in an amount from about 100 to about 25,000 ppm of the composition and the at least one sweet taste improving polyamino acid additive may be present in an amount from about 30 to about 2,000 ppm of the composition. Non-limiting examples include at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with glycine and poly-L-α-lysine; and at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame, potassium or other salts, or neotame, in combination with glycine and poly-L-ε-lysine.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving amino acid additive and at least one sweet taste improving organic acid additive is provided. The at least one sweet taste improving amino acid additive may be present in an amount from about 100 to about 25,000 ppm of the composition and the at least one sweet taste improving organic acid additive may be present in an amount from about 10 to about 5,000 ppm of the composition. A non-limiting example includes at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with glycine and sodium gluconate.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving amino acid additive and at least one sweet taste improving carbohydrate additive is provided. The at least one sweet taste improving amino acid additive may be present in an amount from about 100 to about 25,000 ppm of the composition and the at least one sweet taste improving carbohydrate additive may be present in an amount from about 1,000 to about 100,000 ppm of the composition. A non-limiting example includes at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with L-alanine and fructose.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving amino acid additive, at least one sweet taste improving polyol additive, at least one sweet taste improving inorganic salt additive, and at least one sweet taste improving organic acid salt additive is provided. The at least one sweet taste improving amino acid additive may be present in an amount from about 100 to about 25,000 ppm of the composition, the at least one sweet taste improving polyol additive may be present in an amount from about 400 to about 80,000 ppm of the composition, the at least one sweet taste improving inorganic salt additive may be present in an amount from about 25 to about 5,000 ppm of the composition, and the at least one sweet taste improving organic acid salt additive may be present in an amount from about 20 to about 10,000 ppm of the composition. A non-limiting example includes at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with erythritol, glycine, KCl, KH₂PO₄, and choline chloride.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving amino acid additive, at least one sweet taste improving carbohydrate additive, and at least one sweet taste improving polyol additive is provided. The at least one sweet taste improving amino acid additive may be present in an amount from about 100 to about 25,000 ppm of the composition, the at least one sweet taste improving carbohydrate additive may be present in an amount from about 1,000 to about 100,000 ppm of the composition, and the at least one sweet taste improving polyol additive may be present in an amount from about 400 to about 80,000 ppm of the composition. A non-limiting example includes at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with L-alanine, fructose, and erythritol.

A functional sweetener composition comprising at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with at least one sweet taste improving amino acid additive, at least one sweet taste improving polyol additive, and at least one sweet taste improving inorganic acid salt additive is provided. The at least one sweet taste improving amino acid additive may be present in an amount from about 100 to about 25,000 ppm of the composition, the at least one sweet taste improving polyol additive may be present in an amount from about 400 to about 80,000 ppm of the composition, and the at least one sweet taste improving inorganic acid salt additive may be present in an amount from about 25 to about 5,000 ppm of the composition. A non-limiting example includes at least one functional ingredient and a sweetener comprising rebaudioside-A (REBA), stevia, stevioside, mogroside IV, mogroside V, Luo Han Guo sweetener, monatin, curculin, sucralose, saccharin, cyclamate, aspartame, acesulfame potassium or other salts, or neotame, in combination with erythritol, glycine, KCl, and KH₂PO₄.

A functional sweetener composition comprising at least one functional ingredient and a sweetener comprising rebaudioside-A (REBA), stevia, stevioside, mogroside IV, mogroside V, Luo Han Guo sweetener, monatin, curculin, glycyrrihizin such as mono-ammonium glycyrrhizic acid salt hydrate, sucralose, saccharin, cyclamate, aspartame, acesulfame potassium or other salts, or neotame, in combination with a sweet taste improving inorganic acid salt additive is provided. A non-limiting example includes at least one functional ingredient and a sweetener comprising rebaudioside-A (REBA), stevia, stevioside, mogroside IV, mogroside V, Luo Han Guo sweetener, monatin, curculin, glycyrrihizin such as mono-ammonium glycyrrhizic acid salt hydrate, sucralose, saccharin, cyclamate, aspartame, acesulfame potassium or other salts, or neotame, in combination with sodium chloride.

A composition is provided comprising at least one functional ingredient and a sweetener composition comprising rebaudioside-A (REBA), stevia, stevioside, mogroside IV, mogroside V, Luo Han Guo sweetener, monatin, curculin, glycyrrihizin such as mono-ammonium glycyrrhizic acid salt hydrate, sucralose, saccharin, cyclamate, aspartame, acesulfame potassium or other salts, or neotame, in combination with at least one sweet taste improving polyol additive and at least one sweet taste improving organic acid additive. Desirably, the at least one sweet taste improving polyol additive is present in an amount from about 20,000 to about 50,000 ppm of the composition and the at least one sweet taste improving organic acid additive is present in an amount from about 10 to about 5,000 ppm of the composition. Wherein more than one sweet taste improving organic acid additive is present in the composition, the plurality of sweet taste improving organic acid additives are present in an amount from about 500 to about 2,500 ppm of the composition, more particularly in an amount from about 500 to about 1,500 ppm of the composition. The composition described hereinabove may further comprise at least one sweet taste improving inorganic acid additive, at least one sweet taste improving inorganic acid salt additive, at least one sweet taste improving organic acid salt additive, or combinations thereof.

A composition comprising at least one functional ingredient and a sweetener composition comprising REBA in combination with at least one sweet taste improving polyol additive and at least one sweet taste improving organic acid additive is provided. Desirably, the REBA has a purity from about 50 to about 100% by weight of REBA, more desirably from about 80 to about 99.5% by weight REBA, most desirably from about 97 to about 99.5% by weight REBA in a steviolglycoside mixture. The REBA may be present in the composition in an amount from about 100 to about 3,000 ppm, more desirably in an amount from about 200 to about 2,000 ppm, and even more desirably in an amount from about 250 to about 750 ppm of the composition. Desirably, the at least one sweet taste improving polyol additive is present in an amount from about 20,000 to about 50,000 ppm of the composition and the at least one sweet taste improving organic acid additive is present in an amount from about 10 to about 5,000 ppm of the composition. The at least one sweet taste improving polyol additive may be present in an amount from about 30,000 to about 40,000 ppm and the at least one sweet taste improving organic acid additive may be present in an amount from about 500 to about 2,500 ppm of the composition. A plurality of sweet taste improving organic acid additives may be present in the sweetener composition in an amount from about 500 to about 2,500 ppm of the composition, the plurality of organic acid additives comprising a mixture of lactic acid in an amount from about 40 to about 250 ppm, citric acid in an amount from about 150 to about 460 ppm, malic acid in an amount from about 150 to about 460 ppm, and tartaric acid in an amount from about 150 to about 460 ppm. A non-limiting example includes REBA in combination with erythritol, lactic acid, citric acid, malic acid, tartaric acid, or combinations thereof. The composition may comprise 34,000 ppm of erythritol, 80 ppm of lactic acid, 310 ppm of citric acid, 310 ppm of malic acid, 310 ppm or tartaric acid, and 550 ppm of REBA. Desirably, the REBA has a purity from about 80 to about 99.5% by weight of REBA, more desirably from about 97 to about 99.5% by weight REBA in a steviolglycoside mixture. The composition optionally also may include flavorants such as caramel, vanilla, or other such flavorants as described herein, or combinations thereof. Such a composition may be a carbonated soft drink, such as a cola, although other types of beverages are contemplated as well. Those of ordinary skill in the art should appreciate that the amounts of the sweet taste improving organic acids in a carbonated beverage may be modified to obtain a pH from about 2.3 to about 3.5. In addition, those of ordinary skill in the art also should appreciate that sweet taste improving inorganic acid additives, such as phorphoric acid, benzoic acid, and sorbic acid, may be used individually or in combination in a carbonated beverage in order to obtain a pH from about 2.3 to about 3.5.

The composition comprising at least one functional ingredient and a sweetener composition comprising REBA in combination with at least one sweet taste improving polyol additive and at least one sweet taste improving organic acid additive described hereinabove may further comprise at least one sweet taste improving inorganic acid additive. Desirably, at least one sweet taste improving inorganic acid additive is present in an amount from about 25 to about 5,000 ppm of the composition. Non-limiting examples of sweet taste improving inorganic acid additives include phosphoric acid, benzoic acid, sorbic acid, and combinations thereof.

The composition comprising at least one functional ingredient and a sweetener composition comprising REBA in combination with at least one sweet taste improving polyol additive and at least one sweet taste improving organic acid additive described hereinabove may further comprise at least one sweet taste improving inorganic acid salt additive and/or at least one sweet taste improving organic acid salt additive. Desirably, the at least one sweet taste improving inorganic acid salt additive is present in an amount from about 25 to about 5,000 ppm of the composition, more desirably in an amount from about 50 to about 250 ppm, most desirably in an amount of about 150 ppm. Desirably, the at least one sweet taste improving organic acid salt additive is present in an amount from about 20 to about 10,000 ppm of the composition, more desirably in an amount from about 50 to about 350 ppm, most desirably in an amount of about 148 ppm. Non-limiting examples include REBA in combination with erythritol, sodium chloride or magnesium chloride, and lactic acid, citric acid, malic acid, tartaric acid, or combinations thereof; REBA in combination with erythritol, potassium citrate or sodium citrate, and lactic acid, citric acid, malic acid, tartaric acid, or combinations thereof; or REBA in combination with erythritol, sodium chloride and sodium citrate, lactic acid, citric acid, malic acid, and tartaric acid, or combinations thereof.

The composition comprising at least one functional ingredient and a sweetener composition comprising REBA in combination with at least one sweet taste improving polyol additive, at least one sweet taste improving inorganic acid additive, and at least one sweet taste improving organic acid additive described hereinabove may further comprise at least one sweet taste improving inorganic acid salt additive and/or at least one sweet taste improving organic acid salt additive. Desirably, the at least one sweet taste improving inorganic acid salt additive is present in an amount from about 25 to about 5,000 ppm of the composition, more desirably in an amount from about 50 to about 250 ppm, most desirably in an amount of about 150 ppm. Desirably, the at least one sweet taste improving organic acid salt additive is present in an amount from about 20 to about 10,000 ppm of the composition, more desirably in an amount from about 50 to about 350 ppm, most desirably in an amount of about 148 ppm. Non-limiting examples include REBA in combination with erythritol, phosphoric acid, sodium chloride or magnesium chloride, and lactic acid, citric acid, malic acid, tartaric acid, or combinations thereof; REBA in combination with erythritol, phosphoric acid, potassium citrate or sodium citrate, and lactic acid, citric acid, malic acid, tartaric acid, or combinations thereof; or REBA in combination with erythritol, phosphoric acid, sodium chloride and sodium citrate, lactic acid, citric acid, malic acid, and tartaric acid, or combinations thereof.

It is contemplated that the combination of at least one natural and/or synthetic high-potency sweetener to at least one sweet taste improving composition may be carried out in any pH range that does not materially or adversely affect the taste of the functional sweetener composition or the functional sweetened composition. A non-limiting example of the pH range may be from about 2 to about 8. A further example includes a pH range from about 2 to about 5.

### IV. Tabletop Functional Sweetener Compositions

The functional sweetener compositions may comprise a tabletop functional sweetener composition comprising at least one natural and/or synthetic high-potency sweetener in combination with: (i) at least one functional ingredient; (ii) at least one bulking agent; and (iii) optionally at least one sweet taste improving composition and/or anti-caking agent with improved temporal and/or flavor profile.

Suitable "bulking agents" include maltodextrin (10 DE, 18 DE, or 5 DE), corn syrup solids (20 or 36 DE), sucrose, fructose, glucose, invert sugar, sorbitol, xylose, ribulose, mannose, xylitol, mannitol, galactitol, erythritol, maltitol, lactitol, isomalt, maltose, tagatose, lactose, inulin, glycerol, propylene glycol, polyols, polydextrose, fructooligosaccharides, cellulose and cellulose derivatives, and the like, and mixtures thereof. Additionally, granulated sugar (sucrose) or other caloric sweeteners such as crystalline fructose, other carbohydrates, or sugar alcohols can be used as a bulking agent due to their provision of good content uniformity without the addition of significant calories. In one embodiment, a bulking agent may be used as a sweet taste improving composition.

As used herein the phrase "anti-caking agent" and "flow agent" refer to any composition which prevents, reduces, inhibits, or suppresses at least one natural and/or synthetic high-potency sweetener molecule from attaching, binding, or contacting to another natural and/or synthetic high-potency sweetener molecule. Alternatively, anti-caking agent may refer to any composition which assists in content uniformity and uniform dissolution. Non-limiting examples of anti-caking agents include cream of tartar, calcium silicate, silicon dioxide, microcrystalline cellulose (Avicel, FMC BioPolymer, Philadelphia, Pennsylvania), and tricalcium phosphate. The anti-caking agents may be present in the tabletop functional sweetener composition in an amount from about 0.001 to about 3 % by weight of the tabletop functional sweetener composition.

Tabletop functional sweetener compositions are embodied and packaged in numerous different forms and it is intended that the tabletop functional sweetener compositions may be of any form known in the art. Non-limiting examples include powder form, granular form, packets, tablets, sachets, pellets, cubes, solids, and liquids.

A tabletop functional sweetener composition comprises a single-serving (portion control) packet comprising a dry-blend of a functional sweetener formulation. Dry-blend formulations generally may comprise powder or granules. Although the tabletop functional sweetener packet may be of any size, an illustrative non-limiting example of conventional portion control tabletop sweetener packets are approximately 2.5 by 1.5 inches and hold approximately 1 gram of a sweetener composition having a sweetness equivalent to 2 teaspoons of granulated sugar (∼ 8 g). The amount of natural and/or synthetic high-potency sweetener in a dry-blend tabletop functional sweetener formulation will vary due to the varying potency of different natural and/or synthetic high-potency sweeteners. A dry-blend tabletop functional sweetener formulation may comprise a natural and/or synthetic high-potency sweetener in an amount from about 1% (w/w) to about 10 % (w/w) of the tabletop functional sweetener composition.

Solid tabletop functional sweetener embodiments include cubes and tablets. A non-limiting example of conventional cubes are equivalent in size to a standard cube of granulated sugar, which is approximately 2.2 x 2.2 x 2.2 cm³ and weigh approximately 8 g. A solid tabletop sweetener may be in the form of a tablet or any other form known to those skilled in the art.

A tabletop functional sweetener composition may also be embodied in the form of a liquid, wherein the NHPS is combined with a liquid carrier. Suitable non-limiting examples of carrier agents for liquid tabletop functional sweeteners include water, alcohol, polyol, glycerin base or citric acid base dissolved in water, and mixtures thereof. Due to the varying potencies of the different high-potency sweeteners, the amount of high-potency sweetener in a liquid tabletop functional sweetener formulation will also vary. The sweetness equivalent of a tabletop functional sweetener composition for any of the forms described herein or known in the art may be varied to obtain a desired sweetness profile. For example, a tabletop functional sweetener composition may comprise a sweetness comparable to that of an equivalent amount of standard sugar. The tabletop functional sweetener composition may comprise a sweetness of up to 100 times that of an equivalent amount of sugar. The tabletop functional sweetener composition may comprise a sweetness of up to 90 times, 80 times, 70 times, 60 times, 50 times, 40 times, 30 times, 20 times, 10 times, 9 times, 8 times, 7 times, 6 times, 5 times, 4 times, 3 times, and 2 times that of an equivalent amount of sugar.

The tabletop functional sweetener composition may also be formulated for targeted uses, for example, in beverage, food, pharmaceutical, cosmetics, herbal/vitamins, tobacco, and in any other products which may be sweetened. For example, a tabletop functional sweetener composition for baking may be formulated having additional protecting agents such as encapsulants. Other forms will be readily apparent to those skilled in the tabletop sweetener art.

Commonly used methods for making powder or granulated functional sweetener formulations for packets include fluid bed agglomeration processes. Other methods for making tabletop sweetener compositions are well known to those of ordinary skill in the art.

Those skilled in the art appreciate that the amount of natural and/or synthetic high-potency sweetener and amount and types of sweet taste improving composition, bulking agent, and/or anti-caking agent can be modified in order to tailor the taste of the tabletop sweetener composition to a desired profile and end use.

Specific embodiments of tabletop sweetener compositions and methods of making tabletop functional sweetener compositions are disclosed in U.S. Provisional Application No. 60/805,209, filed on June 19, 2006, by DuBois, et al., the disclosure of which is incorporated herein by reference in its entirety.

### V. Orally Ingestible Compositions

As used herein, "orally ingestible composition" and "sweetenable composition" are synonymous and mean substances which are contacted with the mouth of man or animal, including substances which are taken into and subsequently ejected from the mouth and substances which are drunk, eaten, swallowed or otherwise ingested, and are safe for human or animal consumption when used in a generally acceptable range. These compositions include food, beverage, pharmaceutical, tobacco, nutraceutical, oral hygienic/cosmetic products, and the like. Non-limiting examples of these products include non-carbonated and carbonated beverages such as colas, ginger ales, root beers, ciders, fruit-flavored soft drinks (e.g., citrus-flavored soft drinks such as lemon-lime or orange), powdered soft drinks, and the like; fruit juices originating in fruits or vegetables, fruit juices including squeezed juices or the like, fruit juices containing fruit particles, fruit beverages, fruit juice beverages, beverages containing fruit juices, beverages with fruit flavorings, vegetable juices, juices containing vegetables, and mixed juices containing fruits and vegetables; sport drinks, energy drinks, near water and the like drinks (e.g., water with natural or synthetic flavorants); tea type or favorite type beverages such as coffee, cocoa, black tea, green tea, oolong tea and the like; beverages containing milk components such as milk beverages, coffee containing milk components, cafe au lait, milk tea, fruit milk beverages, drinkable yogurt, lactic acid bacteria beverages or the like; dairy products; bakery products; desserts such as yogurt, jellies, drinkable jellies, puddings, Bavarian cream, blancmange, cakes, brownies, mousse and the like, sweetened food products eaten at tea time or following meals; frozen foods; cold confections, e. g. types of ice cream such as ice cream, ice milk, lacto-ice and the like (food products in which sweeteners and various other types of raw materials are added to milk products, and the resulting mixture is agitated and frozen), and ice confections such as sherbets, dessert ices and the like (food products in which various other types of raw materials are added to a sugary liquid, and the resulting mixture is agitated and frozen); ice cream; general confections, e. g., baked confections or steamed confections such as cakes, crackers, biscuits, buns with bean-jam filling and the like; rice cakes and snacks; table top products; general sugar confections such as chewing gum (e.g. including compositions which comprise a substantially water-insoluble, chewable gum base, such as chicle or substitutes thereof, including jetulong, guttakay rubber or certain comestible natural synthetic resins or waxes), hard candy, soft candy, mints, nougat candy, jelly beans and the like; sauces including fruit flavored sauces, chocolate sauces and the like; edible gels; crèmes including butter crèmes, flour pastes, whipped cream and the like; jams including strawberry jam, marmalade and the like; breads including sweet breads and the like or other starch products; spice; general condiments including seasoned soy sauce used on roasted meats, roast fowl, barbecued meat and the like, as well as tomato catsup, sauces, noodle broth and the like; processed agricultural products, livestock products or seafood; processed meat products such as sausage and the like; retort food products, pickles, preserves boiled in soy sauce, delicacies, side dishes; snacks such as potato chips, cookies, or the like; cereal products; drugs or quasi-drugs that are administered orally or used in the oral cavity (e.g., vitamins, cough syrups, cough drops, chewable medicine tablets, amino acids, bitter-tasting drug or pharmaceutical agents, acidulants or the like), wherein the drug may be in solid, liquid, gel, or gas form such as a pill, tablet, spray, capsule, syrup, drop, troche agent, powder, and the like; personal care products such as other oral compositions used in the oral cavity such as mouth freshening agents, gargling agents, mouth rinsing agents, toothpaste, tooth polish, dentrifices, mouth sprays, teeth-whitening agents and the like; dietary supplements; tobacco products including smoke and smokeless tobacco products such as snuff, cigarette, pipe and cigar tobacco, and all forms of tobacco such as shredded filler, leaf, stem, stalk, homogenized leaf cured, reconstituted binders and reconstituted tobacco from tobacco dust, fines or ether sources in sheet, pellet or other forms, tobacco substitutes formulated from non-tobacco materials, dip or chewing tobacco; animal feed; and nutraceutical products, which includes any food or part of a food that may provide medicinal or health benefits, including the prevention and treatment of disease (e.g., cardiovascular disease and levels of high cholesterol in the blood, diabetes, osteoporosis, inflammation, or autoimmune disorders).

Generally, the amount of natural and/or synthetic high-potency sweetener present in a sweetened composition varies widely depending on the particular type of sweetened composition and its desired sweetness. Those of ordinary skill in the art can readily discern the appropriate amount of sweetener to put in the sweetened composition. The at least one natural and/or synthetic high-potency sweetener may be present in the sweetened composition in an amount in the range of about 1 to about 5,000 ppm of the sweetened composition and the at least one sweet taste improving composition is present in the sweetened composition in an amount in the range of about 0.1 to about 100,000 ppm of the sweetened composition.

Suitable amounts of natural high-potency sweeteners for sweetenable compositions may comprise amounts in the range from about 100 ppm to about 3,000 ppm for rebaudioside A; from about 50 ppm to about 3,000 ppm for stevia; from about 50 ppm to about 3,000 ppm for stevioside; from about 50 ppm to about 3,000 ppm for mogroside IV; from about 50 ppm to about 3,000 ppm for mogroside V; from about 50 ppm to about 3,000 ppm for Luo Han Guo sweetener; from about 5 ppm to about 300 ppm for monatin, from about 5 ppm to about 200 ppm for thaumatin; and from about 50 ppm to about 3,000 ppm for mono-ammonium glycyrrhizic acid salt hydrate.

Suitable amounts of synthetic high-potency sweeteners for sweetenable compositions may comprise a range from about 1 ppm to about 60 ppm for alitame; from about 10 ppm to about 600 ppm for aspartame; from about 1 ppm to about 20 ppm for neotame; from about 10 ppm to about 500 ppm for acesulfame potassium; from about 50 ppm to about 5,000 ppm for cyclamate; from about 10 ppm to about 500 ppm for saccharin; from about 5 ppm to about 250 ppm for sucralose; from about 1 ppm to about 20 ppm for N-[N-[3-(3-hydroxy-4-methoxyphenyl)propyl]-L-α-aspartyl]-L-phenylalanine 1-methyl ester; from about I ppm to about 20 ppm for N-[N-[3-(3-hydroxy-4-methoxyphenyl)-3-methylbutyl]-L-α-aspartyl]-L-phenylalanine 1-methyl ester; and from about 1 ppm to about 20 ppm for N-[N-[3-(3-methoxy-4-hydroxyphenyl)propyl]-L-α-aspartyl]-L-phenylalanine 1-methyl ester.

An orally ingestible composition may comprise a carbonated beverage comprising at least one natural and/or synthetic high-potency sweetener, at least one sweet taste improving composition, and at least one functional ingredient; wherein the at least one natural and/or synthetic high-potency sweetener comprises rebaudioside A, rebaudioside B, rebaudioside C, rebaudioside D, rebaudioside E, rebaudioside F, dulcoside A, dulcoside B, rubusoside, stevia, stevioside, mogroside IV, mogroside V, Luo Han Guo sweetener, siamenoside, monatin and its salts (monatin SS, RR, RS, SR), curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, cyclocarioside I, sucralose, acesulfame potassium or other salts, aspartame, alitame, saccharin, neohesperidin dihydrochalcone, cyclamate, neotame, N-[N-[3-(3-hydroxy-4-methoxyphenyl)propyl]-L-α-aspartyl]-L-phenylaianine 1-methyl ester, N-[N-[3-(3-hydroxy-4-methoxyphenyl)-3-methylbutyl]-L-α-aspartyl]-L-phenylalanine 1-methyl ester, N-[N-[3-(3-methoxy-4-hydroxyphenyl)propyl]-L-α-aspartyl]-L-phenylalanine 1-methyl ester, salts thereof, or combinations thereof; wherein the at least one sweet taste improving composition is selected from the group consisting of carbohydrates, polyols, amino acids and their corresponding salts, polyamino acids and their corresponding salts, sugar acids and their corresponding salts, organic acids, inorganic acids, organic salts, inorganic salts, bitter compounds, flavorants, astringent compounds, polymers, proteins or protein hydrolysates, surfactants, emulsifiers, flavonoids, alcohols, and combinations thereof; and wherein the at least one functional ingredient comprises at least one agent for the treatment and/or prevention of autoimmune disorders. Specific combinations of sweet taste improving compositions are disclosed in U.S. Provisional Application Nos. 60/739,302 and 60/739,124.

The at least one functional ingredient may require special processing in order to be incorporated into the functional sweetened composition. This is particularly relevant when the functional sweetened composition is aqueous and the at least one functional ingredient is hydrophobic. Techniques of incorporating hydrophobic compositions into aqueous solutions are well known to those of ordinary skill in the art, non-limiting examples of which include homogenization, encapsulation, emulsions, and addition of stabilizers, gums, and the like.

The process for producing a substantially stable dispersion of the at least one functional ingredient in an aqueous functional sweetened composition may comprise mixing the at least one functional ingredient with the aqueous orally ingestible composition to form a first dispersion of particles, heating the first dispersion of particles, and homogenizing the heated first dispersion particles to obtain an aqueous functional sweetened composition comprising particles of the at least one functional ingredient ranging in size from about 0.1 micron to about 50 microns. This method is disclosed further in U.S. Application Nos. 10/458,692 and 11/315,206, filed on October 24, 2003, and December 23, 2005, respectively, the disclosures of which are incorporated herein by reference in their entirety.

The functional sweetener compositions and orally ingestible compositions containing the same are useful for providing healthy benefits beyond basic nutrition. For example, such benefits may include the treatment and/or prevention of autoimmune disorders.

Unless otherwise specified, %'s are by weight.

### Example Set A

### Example A1

A rebaudioside A diet cola beverage (sweetness level 10 % sucrose equivalent) is prepared with 30 mg of an omega-3 fatty acid per serving (∼ 240 mL), 60 I.U. of vitamin D3 per serving, 400 ppm of rehaudioside A, and 3.5 % erythritol.

### Example A2

A rebaudioside A diet lemon-lime beverage (sweetness level 10 % sucrose equivalent) is prepared with 30 mg of an omega-3 fatty acid per serving (∼ 240 mL), 60 I.U. of vitamin D3 per serving, 400 ppm of rebaudioside A, and 3.5 % erythritol.

The following Examples B1-B3, C1-C3, D, and E1-E3 illustrate methods of making purified rebaudioside A:

### Example Set B

**Table 2: Summary of Examples B1-3**

| | **Crude Rebaudioside A (g)** | **Ethanol (95%)(mL)** | **Solvent Methanol (99%)(mL)** | **Water (mL)** | **Heating T (°C)** | **Drying T (°C)** | **Yield (g)** | **HPLC Purity (wt/wt %)** |
|---|---|---|---|---|---|---|---|---|
| B1 | 400 | 1200 | 400 | 320 | 50 | 50 | 130 | 98.9 |
| B2 | 100 | 320 | 120 | 50 | 30-40 | 60 | 72 | 98.3 |
| B3 | 50 | 160 | 60 | 25 | ∼30 | 60 | 27.3 | 98.2 |

### Example B1

Crude rebaudioside A (77.4% purity) mixture was obtained from a commercial source. The impurities (6.2% stevioside, 5.6% rebaudioside C, 0.6 % rebauiodioside F, 1.0 % other steviolglycosides, 3.0% rebaudioside D, 4.9% rebaudioside B, 0.3% steviolbioside) were identified and quantified using HPLC on dry basis, moisture content 4.7%.

Crude rebaudioside A (400 g), ethanol (95%, 1200 mL), methanol (99%, 400 mL) and water (320 mL) were combined and heated to 50°C for 10 minutes. The clear solution was cooled to 22°C for 16 hours. The white crystals were filtered and washed twice with ethanol (2 x 200 mL, 95%) and dried in a vacuum oven at 50°C for 16-24 hours under reduced pressure (20 mm).

The final composition of substantially pure rebaudioside A (130 g) comprised 98.91% rebaudioside A, 0.06% stevioside, 0.03% rebaudioside C, 0.12% rebaudioside F, 0.13% other steviolglycosides, 0.1% rebaudioside D, 0.49% rebaudioside B and 0.03% steviolbioside, all by weight.

### Example B2

Crude rebaudioside A (80.37 %) was obtained from a commercial source. The impurities (6.22% stevioside, 2.28% rebaudioside C, 0.35% Dulcoside, 0.78 % rebaudioside F, 0.72 % other steviolglycosides, 3.33% rebaudioside B, 0.07% steviolbioside) were identified by HPLC on dry basis, moisture content 3.4%.

Crude rebaudioside A (100 g), ethanol (95%, 320 mL), methanol (99%, 120 mL) and water (50 mL) were combined and heated to 30-40°C for 10 minutes. The clear solution was cooled to 22°C for 16 hours. The white crystals were filtered and washed twice with ethanol (2 x 50 mL, 95%). The wet filter cake (88 g) was slurried in ethanol (95%, 1320 mL) for 16 hours, filtered, washed with ethanol (95%, 2 x 100 mL) and dried in a vacuum oven at 60°C for 16-24 hours under reduced pressure (20 mm).

The final composition of substantially pure rebaudioside A (72 g) comprised 98.29% rebaudioside A, 0.03% stevioside, 0.02% rebaudioside C, 0.17% rebaudioside F, 0.06% rebaudioside D and 1.09% rebaudioside B. Steviolbioside was not detected by HPLC.

### Example B3

Crude rebaudioside A (80.37%) was obtained from a commercial source. The impurities (6.22% stevioside, 2.28% rebaudioside C, 0.35% Dulcoside, 0.78 % rebaudioside F, 0.72 % other steviolglycosides, 3.33% rebaudioside B, 0.07% steviolbioside) were identified by HPLC on dry basis, moisture content 3.4%.

Crude rebaudioside A (50 g), ethanol (95%, 160 mL), methanol (99%, 60 mL) and water (25 mL) were combined and heated to approximately 30°C for 10 minutes. The clear solution was cooled to 22°C for 16 hours. The white crystals were filtered and washed twice with ethanol (2 x 25 mL, 95%). The wet filter cake (40 g) was slurried in methanol (99%, 600 mL) for 16 hours, filtered, washed with methanol (99%, 2 x 25 mL) and dried in a vacuum oven at 60°C for 16-24 hours under reduced pressure (20 mm).

The final composition of substantially pure rebaudioside A (27.3g) comprised 98.22% rebaudioside A, 0.04% stevioside, 0.04% rebaudioside C, 0.18% rebaudioside F, 0.08% rebaudioside D and 1.03% rebaudioside B. Steviolbioside was not detected by HPLC.

### Example Set C

**Table 3: Summary of Examples C1-3**

| | | **Solvent** | | | | | |
|---|---|---|---|---|---|---|---|
| | **Crude Rebaudioside A (g)** | **Ethanol (95%)(mL)** | **Organic Co-solvent (mL)** | **Water (mL)** | **Wash Solvent** | **Yield (g)** | **HPLC Purity (%)** |
| C1 | 5 | 15 | Methanol (6) | 3.5 | EtOH/MeOH (3:1 v/v) | 2.6 | >99 |
| C2 | 5 | 15 | Methanol (5) | 4 | EtOH/MeOH (3:1 v/v) | 2.3 | >99 |
| C3 | 5 | 16 | Methanol (6) | 2.5 | *EtOH/MeOH (8:3 v/v) | 3.2 | >98 |

### Example C1

A mixture of crude rebaudioside A (80.37 % purity, 5 g), ethanol (95%, 15 mL), methanol (5 mL) and water (3.5 mL) were combined and heated to reflux for 10 minutes. The clear solution was cooled to 22°C for 16 hours while stirring. The white crystalline product was filtered, washed twice with ethanol:methanol (5.0 mL, 3:1, v/v) mixture and dried in a vacuum oven at 50°C for 16-24 hours under reduced pressure (20 mm) to yield 2.6 g of purified product (>99% by HPLC).

### Example C2

A mixture of crude rebaudioside A (80.37 % purity, 5 g), ethanol (95%, 15 mL), methanol (5 mL) and water (4.0 mL) were combined and heated to reflux for 10 minutes. The clear solution was cooled to 22°C for 16 hours while stirring. The white crystalline product was filtered, washed twice with ethanol:methanol (5.0 mL, 3:1, v/v) mixture and dried in a vacuum oven at 50°C for 16-24 hours under reduced pressure (20 mm) to yield 2.3 g of purified product (>99% by HPLC).

### Example C3

A mixture of crude rebaudioside A (80.37 % purity, 5 g), ethanol (95%, 16 mL), methanol (6 mL) and water (2.5 mL) were combined and heated to reflux for 10 minutes. The clear solution was cooled to 22°C for 2 hours. During this time, crystals started to appear. The mixture is stirred at room temperature for 16 hours. The white crystalline product was filtered, washed twice with ethanol:methanol (5.0 mL, 8:3, v/v) mixture and dried in a vacuum oven at 50°C for 16-24 hours under reduced pressure (20 mm) to yield 3.2 g of purified product (>98% by HPLC).

### Example D

**Table 4: Summary of Example D**

| | | **Solvent** | | | | |
|---|---|---|---|---|---|---|
| | **Crude Rebaudioside A (g)** | **Organic Solvent (mL)** | **Water (mL)** | **Wash Solvent** | **Yield (g)** | **HPLC Purity (%)** |
| D | 50 | EtOH (160) | 40 | EtOH | 19.8 | 99.5 |

A mixture of crude rebaudioside A (80.37 % purity, 50 g), ethanol (95%, 160 mL) and water (40 mL) were combined and heated to reflux for 30 minutes. The mixture was then allowed to cool to ambient temperature for 16-24 hours. The white crystalline product was filtered, washed twice with ethanol (95%, 25 mL), and dried in a vacuum oven at 60°C for 16-24 hours under reduced pressure (20 mm) to yield 19.8 g of purified product (99.5% by HPLC).

### Example E

**Table 5: Summary of Examples E1-3**

| | **Crude Rebaudioside A (g)** | **Ethanol (95%)(mL)** | **Organic Co-solvent (mL)** | **Water (mL)** | **Methanol Slurry (mL)** | **Yield (g)** | **HPLC Purity (%)** |
|---|---|---|---|---|---|---|---|
| E1 | 50 | 160 | Methanol (60) | 25 | 200 | 12.7 | >97 |
| E2 | 50 | 160 | Methanol (60) | 25 | 300 | 18.6 | >97 |
| E3 | 50 | 160 | Methanol (60) | 25 | 350 | 22.2 | >97 |

### Example E1

A mixture of crude rebaudioside A (41% purity, 50 g), ethanol (95%, 160 mL), methanol (99.8 %, 60 mL) and water (25 mL) were combined by stirring at 22°C. A white product crystallized out in 5-20 hours. The mixture was stirred for additional 48 hours. The white crystalline product was filtered and washed twice with ethanol (95%, 25 mL). The wet cake of white crystalline product then was slurried in methanol (99.8 %, 200 mL) for 16 hours, filtered, washed twice with methanol (99.8 %, 25 mL), and dried in a vacuum oven at 60°C for 16-24 hours under reduced pressure (20 mm) to give 12.7 g of purified product (>97% by HPLC).

### Example E2

A mixture of crude rebaudioside A (48% purity, 50 g), ethanol (95%, 160 mL), methanol (99.8 %, 60 mL) and water (25 mL) was combined by stirring at 22°C. The white product crystallized out in 3-6 hours. The mixture was stirred for additional 48 hours. The white crystalline product was filtered and washed twice with ethanol (95%, 25 mL). The wet cake of white crystalline product then was slurried in methanol (99.8 %, 300 mL) for 16 hours, filtered, washed twice with methanol (99.8 %, 25 mL) and dried in a vacuum oven at 60°C for 16-24 hours under reduced pressure (20 mm) to give 18.6 g of purified product (>97% by HPLC).

### Example E3

A mixture of crude rebaudioside A (55% purity, 50 g), ethanol (95%, 160 mL), methanol (99.8 %, 60 mL) and water (25 mL) was combined by stirring at 22°C. The white product crystallized out in 15-30 minutes. The mixture was stirred for an additional 48 hours. The white crystalline product was filtered and washed twice with ethanol (95%, 25 mL). The wet cake of white crystalline product was slurried in methanol (99.8 %, 350 mL) for 16 hours, filtered, washed twice with methanol (99.8 %, 25 mL) and dried in a vacuum oven at 60°C for 16-24 hours under reduced pressure (20 mm) to give 22.2 g of purified product (>97% by HPLC).

### Example F

A solution of rebaudioside A ( >97% pure by HPLC ) was prepared in double distilled water (12.5 gm in 50 mL, 25 % concentration) by stirring the mixture at 40°C for 5 minutes. An amorphous rebaudioside A polymorph was formed by immediately using the clear solution for spray drying with the Lab-Plant spray drier SD-04 instrument (Lab-Plant Ltd., West Yorkshire, U.K.). The solution was fed through the feed pump into the nozzle atomizer which atomized it into a spray of droplets with the help of a constant flow of nitrogen / air. Moisture was evaporated from the droplets under controlled temperature conditions (about 90 to about 97°C) and airflow conditions in the drying chamber and resulted in the formation of dry particles. This dry powder (11-12 g, H₂O 6.74 %) was discharged continuously from the drying chamber and was collected in a bottle. The solubility in water at room temperature was determined to be > 35.0 %.

### Example Set G

Sensory evaluation of the samples prepared in Example Set G was carried out under the following protocol, similar to that described hereinabove. In this test protocol, none of the samples were swallowed. All samples were expectorated and the mouth was rinsed with water after the tasting. Immediately upon sensing maximal (sweetness, the sample was expectorated, the mouth was rinsed with water and the rate of sweetness decay ("Sweetness Linger") was measured, where attention was focused on the sweetness 3-4 min after the water rinse. After sample tasting was complete, a salty oyster cracker was chewed followed by a water rinse, and at least 5 minutes followed before tasting the next sample. The sweetness linger was rated by a panel of experts in the sensory evaluation of foods and beverages using the following scale: 0 = no sweetness linger, 1 = very slight sweetness linger, 2 = slight sweetness linger, 3 = moderate sweetness linger, 4 = moderately high sweetness linger, 5 = high sweetness linger.

The "Sweetness Linger" rating for sucrose observed by this protocol is defined as 0. The Sweetness Linger of a 500 ppm of REBA control sample is defined as 5. Experimental samples were tasted by the same protocol, always allowing sufficient time between samples to ensure re-equilibration of the sensory system. Re-tasting of control samples during the course of the experiment was allowed and encouraged.

The comparison taste test was performed between two controls and addition of sweet taste improving additive on the onset and/or sweetness linger.

### Control Samples

REBA is a natural non-caloric sweetener with a very clean flavor profile (i.e., only sweet) and an acceptable sweetness onset rate but with a sweetness which lingers quite noticeably more than that of carbohydrate sweeteners.

The effects of formulation change on the sweetness linger of 400 ppm REBA (equivalent to 8 g sucrose) in a citric acid/potassium citrate composition equivalent to that in a diet lemon-lime beverage were evaluated. The sweetness linger rating of this solution was determined to be 5.

8 g of sugar was dissolved in 100 ml of citrate buffer. The sweetness linger rating of this control sample was determined to be 0.

The following Examples G 1-51 (all not in accordance with the invention) illustrate combinations of rebaudioside A and sweet taste improving compositions:

### Example Set H

Sweet taste improving compositions were combined with a REBA solution to determine their effect on sweetness linger. Screening of the initial sample, or further dilutions, allowed identification of concentrations which were just above-threshold, herein defined as "near-threshold concentrations." The near-threshold additive concentrations, a 6- to 100-fold higher higher additive concentration (depending on the off-taste intensity), and a mid-level additive concentration (halfway between the near-threshold and higher additive concentration) were evaluated to determine the effect on sweetness linger of a REBA solution.

Formulations of a 500 ppm REBA in a phosphoric acid solution (75%) at a pH of 2.5 with phosphoric acid or a pH of 3.1 with citric acid and potassium citrate were prepared prior to the addition of the additives at the three levels of concentration.

Sensory evaluation using the protocol described in Example Set G then was used to evaluate the sweetness linger of the REBA solutions.

### Controls

500 ppm of REBA was dissolved in one liter of carbon-treated water and phosphoric acid (75%) was added until a pH between 2.4 and 2.5 was reached. The sweetness linger rating of this control sample was determined to be 5.

10 g of sugar was dissolved in 100 ml of carbon treated water and phosphoric acid (75%) was added until a pH between 2.4 and 2.5 was reached. The sweetness linger rating of this control sample was determined to be 0.

The following Examples H 1-41 (all not in accordance with the invention) illustrate combinations of rebaudioside A and sweet taste improving compositions:

### Example H38

500 ppm of REBA was dissolved in one liter carbon-treated water and phosphoric acid (75%) was added until a pH between pH 2.4 and 2.5 was reached. 35,000 ppm of erythritol, 3,750 ppm of glycine, 450 ppm of KC1, 680 ppm of KH₂PO₄, and 1,175 ppm of choline chloride were then mixed with the base solution. The sweetness linger of this solution was determined to be 1. This formulation was found to have sugar-like taste characteristics.

### Example H39

500 ppm of REBA was dissolved in one liter carbon-treated water and phosphoric acid (75%) was added until a pH between pH 2.4 and 2.5 was reached. 2,500 ppm of L-alanine, 5,000 ppm of fructose, and 35,000 ppm of erythritol were then mixed with the base solution. The sweetness linger of this solution was determined to be 4. This formulation was found to have sugar-like taste characteristics.

### Example H40

500 ppm of REBA was dissolved in one liter carbon-treated water and phosphoric acid (75%) was added until a pH between pH 2.4 and 2.5 was reached. 35,000 ppm of erythritol, 3,750 ppm of glycine, 450 ppm of KC1, and 680 ppm of KH₂PO₄ were then mixed with the base solution. The sweetness linger of this solution was determined to be 4. This formulation was found to have sugar-like taste characteristics.

### Example H41

360 ppm of REBA was dissolved in one liter carbon-treated water and phosphoric acid (75%) was added until a pH between pH 2.4 and 2.5 was reached. 400 ppm of Fibergum and 35,000 ppm of erythritol were then mixed with the base solution. The sweetness linger of this solution was determined to be 2.

## Claims

1. A functional sweetener composition comprising at least one functional ingredient, rebaudioside A having a purity of 50% to 100% rebaudioside A by weight on a dry basis and erythritol, wherein:
the rebaudioside A is present in an amount ranging from 100 ppm to 3,000 ppm of the functional sweetener composition;
the erythritol is present in an amount ranging from 5,000 ppm to 40,000 ppm of the functional sweetener composition; and
the at least one functional ingredient comprises at least one agent for the treatment and/or prevention of autoimmune disorders.

2. A functional sweetened composition comprising at least one sweetenable composition and a functional sweetener composition, wherein the functional sweetener composition comprises at least one functional ingredient, rebaudioside A having a purity of 50% to 100% rebaudioside A by weight on a dry basis and erythritol, wherein:
the rebaudioside A is present in an amount ranging from 100 ppm to 3,000 ppm of the functional sweetener composition;
the erythritol is present in an amount ranging from 5,000 ppm to 40,000 ppm of the functional sweetener composition; and
the at least one functional ingredient comprises at least one agent for the treatment and/or prevention of autoimmune disorders.

3. A method for imparting a more sugar-like temporal profile, more sugar-like flavor profile, or both to a functional sweetener composition or a functional sweetened composition, comprising combining at least one functional ingredient and rebaudioside A having a purity of 50% to 100% rebaudioside A by weight on a dry basis with erythritol, wherein:
the rebaudioside A is present in an amount ranging from 100 ppm to 3,000 ppm of the functional sweetener composition;
the erythritol is present in an amount ranging from 5,000 ppm to 40,000 ppm of the functional sweetener composition; and
the at least one functional ingredient comprises at least agent for the treatment and/or prevention of autoimmune disorders; the functional sweetened composition further comprising a sweetenable composition.

4. The functional sweetener composition of claim 1, functional sweetened composition of claim 2 or the method of claim 3, wherein the rebaudioside A has a purity greater than 95% up to 100% rebaudioside A by weight on a dry basis.

5. The functional sweetener of claim 1, the functional sweetened composition of claim 2 or the method of claim 3, wherein the erythritol is present in an amount ranging from 10,000 ppm to 35,000 ppm of the functional sweetener composition.

6. The functional sweetener composition of claim 1 or the functional sweetened composition of claim 2 or the method of claim 3,
wherein the at least one agent for the treatment and/or prevention of autoimmune disorders is selected from the group consisting of at least one probiotic, at least one prebiotic, at least one fatty acid, at least one antioxidant, at least one vitamin, or combinations thereof,
wherein the at least one probiotic is selected from the group consisting of a bacteria selected from the genus *Bacillus, Lactobacillus, Bifodobacterium,* and combinations thereof,
wherein the at least one prebiotic is selected from the group consisting of fructooligosaccharides, inulins, isomalto-oligosaccharides, lactilols, lactosucroses, lactuloses, pyrodextrins, soy oligosaccharides, transgalacto-oligosaccharides, xylo-oligosaccharides, or combinations thereof,
wherein the at least one fatty acid comprises an omega-3-fatty acid,
wherein the at least one antioxidant is selected from the group consisting of vitamin cofactors, minerals, hormones, carotenoids, carotenoid terpenoids, non-carotenoid terpenoids, flavonoid polyphenolics, flavonols, flavones, phenols, polyphenols, esters of phenols, esters of polyphenols, nonflavonoid phenolics, isothiocyanates, or combinations thereof.

7. A functional beverage comprising the functional sweetened composition of claim 1,
wherein the functional beverage is selected from the group consisting of a noncarbonated beverage, carbonated beverage, cola, root beer, fruit-flavored beverage, citrus flavored beverage, fruit juice, fruit-containing beverage, vegetable juice, vegetable containing beverage, tea, coffee, dairy beverage, sports drink, energy drink, and flavored water.

## Patentansprüche

1. Funktionale Süßmittelzusammensetzung, umfassend mindestens einen funktionalen Bestandteil, Rebaudiosid-A mit einer Reinheit von 50 Gew.-% bis 100 Gen.-% Rebaudiosid-A auf Trockenbasis und Erythrit, wobei
das Rebaudiosid-A in einer Menge zwischen 100 ppm und 3.000 ppm der funktionalen Süßmittelzusammensetzung vorliegt;
das Erythrit in einer Menge zwischen 5.000 ppm und 40.000 ppm der funktionalen Süßmittelzusammensetzung vorliegt; und
der mindestens eine funktionale Bestandteil mindestens einen Wirkstoff zur Behandlung und/oder Vorbeugung von Autoimmunerkrankungen umfasst.

2. Funktionale gesüßte Zusammensetzung, umfassend mindestens eine süßbare Zusammensetzung und eine funktionale Süßmittelzusammensetzung, wobei die funktionale Süßmittelzusammensetzung mindestens einen funktionalen Bestandteil, Rebaudiosid-A mit einer Reinheit von 50 Gew.-% bis 100 Gew.-% Rebaudiosid-A auf Trockenbasis und Erythrit umfasst, wobei:
das Rebaudiosid-A in einer Menge zwischen 100 ppm und 3.000 ppm der funktionalen Süßmittelzusammensetzung vorliegt;
das Erythrit in einer Menge zwischen 5.000 ppm und 40.000 ppm der funktionalen Süßmittelzusammensetzung vorliegt; und
der mindestens eine funktionale Bestandteil mindestens einen Wirkstoff zur Behandlung und/oder Vorbeugung von Autoimmunerkrankungen umfasst.

3. Verfahren zum Verleihen eines mehr zuckerartigen vorübergehenden Profils, eines mehr zuckerartigen Geschmacksprofils oder beidem für eine funktionale Süßmittelzusammensetzung oder eine funktionale gesüßte Zusammensetzung, umfassend das Kombinieren von mindestens einem funktionalen Bestandteil und Rebaudiosid-A mit einer Reinheit von 50 bis 100 Gew.-% Rebaudiosid A auf Trockenbasis mit Erythrit, wobei:
das Rebaudiosid-A in einer Menge zwischen 100 ppm und 3.000 ppm der funktionalen Süßmittelzusammensetzung vorlegt;
das Erythrit in einer Menge zwischen 5.000 ppm und 40.000 ppm der funktionalen Süßmittelzusammensetzung vorliegt; und
der mindestens eine funktionale Bestandteil mindestens einen Wirkstoff für die Behandlung und/oder Vorbeugung von Autoimmunerkrankungen umfasst; wobei die funktionale gesüßte Zusammensetzung ferner eine süßbare Zusammensetzung umfasst.

4. Funktionale Süßmittelzusammensetzung nach Anspruch 1, funktionale gesüßte Zusammensetzung nach Anspruch 2 oder Verfahren nach Anspruch 3, wobei das Rebaudiosid-A eine Reinheit von mehr als 95 Gew.-% bis zu 100 Gew.-% Rebaudiosid-A auf Trockenbasis besitzt.

5. Funktionales Süßmittel nach Anspruch 1, funktionale gesüßte Zusammensetzung nach Anspruch 2 oder Verfahren nach Anspruch 3, wobei das Erythrit in einer Menge in dem Bereich von 10.000 ppm bis 35.000 ppm der funktionalen Süßmittelzusammensetzung vorliegt.

6. Funktionale Süßmittelzusammensetzung nach Anspruch 1 oder funktionale gesüßte Zusammensetzung nach Anspruch 2 oder Verfahren nach Anspruch 3,
wobei der mindestens eine Wirkstoff für die Behandlung und/oder Vorbeugung von Autoimmunerkrankungen ausgewählt ist aus der Gruppe, bestehend aus mindestens einem Probiotikum, mindestens einem Präbiotikum, mindestens einer Fettsäure, mindestens einem Antioxidationsmittel, mindestens einem Vitamin oder Kombinationen daraus,
wobei das mindestens eine Probiotikum ausgewählt ist aus der Gruppe, bestehend aus Bakterien, ausgewählt aus dem Geschlecht Bacillus, Lactobacillus, Bifidobakterium und Kombinationen davon,
wobei das mindestens eine Präbiotikum ausgewählt ist aus der Gruppe, bestehend aus Fructo-Oligosacchariden, Inulinen, Isomaltooligosacchariden, Lactilolen, Lactosucrosen, Lactulosen, Pyrodextrinen, Sojaoligosacchariden, Transgalactooligosacchariden, Xylooligosacchariden oder Kombinationen davon,
wobei die mindestens eine Fettsäure eine Omega-3-Fettsäure umfasst, wobei das mindestens eine Antioxidationsmittel ausgewählt ist aus der Gruppe, bestehend aus Vitamin-Cofaktoren, Mineralien, Hormonen, Carotinoiden, Caretinoidterpenoiden, nicht caretinoiden Terpenoiden, Flavonoid-Polyphenolen, Flavonolen, Flavonen, Phenolen, Polyphenolen, Estern von Phenolen, Estern von Polyphenolen, nicht flavonoiden Phenolen, Isothiocyanaten oder Kombinationen davon.

7. Funktionales Getränk, umfassend die funktionale gesüßte Zusammensetzung nach Anspruch 1, wobei das funktionale Getränk ausgewählt ist aus der Gruppe, bestehend aus stillem Getränk, Getränk mit Kohlensäure, Cola, Rootbeer, fruchtigen Getränken, Getränken mit Zitronengeschmack, Fruchtsaft, fruchthaltigen Getränken, Gemüsesaft, gemüsehaltigen Getränken, Tee, Kaffee, Milchgetränken, Sportgetränken, Energy-Drinks und aromatisiertem Wasser.

## Revendications

1. Composition d'édulcorant fonctionnelle comprenant au moins un composant fonctionnel, le rébaudioside A, ayant une pureté de 50 % à 100 % de rébaudioside A en poids sur une base sèche et de l'érythrytol, dans laquelle :
le rébaudioside A est présent en une quantité dans la plage de 100 ppm à 3 000 ppm de la composition d'édulcorant fonctionnelle ;
l'érythrytol est présent en une quantité dans la plage de 5 000 ppm à 40 000 ppm de la composition d'édulcorant fonctionnelle ; et
l'au moins un composant fonctionnel comprend au moins un agent pour le traitement et/ou la prévention de troubles auto-immuns.

2. Composition édulcorée fonctionnelle comprenant au moins une composition édulcorable et une composition d'édulcorant fonctionnelle, la composition d'édulcorant fonctionnelle comprenant au moins un composant fonctionnel, le rébaudioside A, ayant une pureté de 50 % à 100 % de rébaudioside A en poids sur une base sèche et de l'érythrytol, dans laquelle :
le rébaudioside A est présent en une quantité dans la plage de 100 ppm à 3 000 ppm de la composition d'édulcorant fonctionnelle ;
l'érythrytol est présent en une quantité dans la plage de 5 000 ppm à 40 000 ppm de la composition d'édulcorant fonctionnelle ; et
l'au moins un composant fonctionnel comprend au moins un agent pour le traitement et/ou la prévention de troubles auto-immuns.

3. Procédé pour conférer un profil temporel plus proche du sucre, un profil gustatif plus proche du sucre, ou les deux à une composition d'édulcorant fonctionnelle ou une composition édulcorée fonctionnelle, comprenant la combinaison d'au moins un composant fonctionnel et de rébaudioside A ayant une pureté de 50 % à 100 % de rébaudioside A en poids sur une base sèche avec de l'érythrytol, dans laquelle :
le rébaudioside A est présent en une quantité dans la plage de 100 ppm à 3 000 ppm de la composition d'édulcorant fonctionnelle ;
l'érythrytol est présent en une quantité dans la plage de 5 000 ppm à 40 000 ppm de la composition d'édulcorant fonctionnelle ; et
l'au moins un composant fonctionnel comprend au moins un agent pour le traitement et/ou la prévention de troubles auto-immuns ; la composition édulcorée fonctionnelle comprenant en outre une composition édulcorable.

4. Composition d'édulcorant fonctionnelle de la revendication 1, composition édulcorée fonctionnelle de la revendication 2 ou procédé de la revendication 3, le rébaudioside A ayant une pureté supérieure à 95 % à 100 % de rébaudioside A en poids sur une base sèche.

5. Édulcorant fonctionnel de la revendication 1, composition édulcorée fonctionnelle de la revendication 2 ou procédé de la revendication 3, l'érythrytol étant présent en une quantité dans la plage de 10 000 ppm à 35 000 ppm de la composition d'édulcorant fonctionnelle.

6. Composition d'édulcorant fonctionnelle de la revendication 1 ou composition édulcorée fonctionnelle de la revendication 2 ou procédé de la revendication 3, dans lesquels l'au moins un agent pour le traitement et/ou la prévention de troubles auto-immuns est choisi dans le groupe constitué d'au moins un probiotique, au moins un prébiotique, au moins un acide gras, au moins un antioxydant, au moins une vitamine, ou des combinaisons de ceux-ci,
l'au moins un probiotique étant choisi dans le groupe constitué d'une bactérie choisie parmi les genres *Bacillus*, *Lactobacillus*, *Bifidojbacterium*, et des combinaisons de ceux-ci,
l'au moins un prébiotique étant choisi dans le groupe constitué de fructo-oligosaccharides, inulines, isomalto-oligosaccharides, lactilols, lactosucroses, lactuloses, pyrodextrines, oligosaccharides de soja, transgalacto-oligosaccharides, xylo-oligosaccharides, ou des combinaisons de ceux-ci,
l'au moins un acide gras comprenant un acide gras oméga 3,
l'au moins un antioxydant étant choisi dans le groupe constitué de cofacteurs de vitamines, minéraux, hormones, caroténoïdes, terpénoïdes caroténoïdes, terpénoïdes non-caroténoïdes, polyphénoliques flavonoïdes, flavonols, flavones, phénols, polyphénols, esters de phénols, esters de polyphénols, phénoliques non-flavonoïdes, isothiocyanates, ou des combinaisons de ceux-ci.

7. Boisson fonctionnelle comprenant la composition édulcorée fonctionnelle de la revendication 1, la boisson fonctionnelle étant choisie dans le groupe constitué d'une boisson non carbonatée, une boisson carbonatée, un cola, une bière de racine, une boisson aromatisée aux fruits, une boisson aromatisée aux agrumes, un jus de fruits, une boisson contenant des fruits, un jus de légumes, une boisson contenant des légumes, du thé, du café, une boisson lactée, une boisson pour sportifs, une boisson énergétique, et de l'eau aromatisée.
